# EUROPEAN PATENT APPLICATION

(11) **EP 4 226 939 A1**
(43) Date of publication of application: **16.08.2023**
(21) Application number: 21877757.1
(22) Date of filing: 08.10.2021
(51) Int. Cl.: A61K 39/39, A61K 39/395, A61K 45/00, A61P 35/00, A61P 35/04, A61P 37/04, A61K 33/243, A61K 35/74, A61K 31/282

(54) **NOVEL USE OF MYCOBACTERIUM TUBERCULOSIS EXTRACT**

(30) Priority: 09.10.2020 JP 2020171491; 25.12.2020 JP 2020217698
(71) Applicant: Zeria Pharmaceutical Co., Ltd., Tokyo 103-8351 (JP)
(72) Inventor: HORII, Takayuki, Tokyo 103-8351 (JP); TANAKA, Takao, Tokyo 103-8351 (JP)
(74) Representative: Engelhard, Markus
(86) International application number: PCT/JP2021/037410
(87) International publication number: WO 2022/075458

(57) **Abstract**

The present disclosure provides a composition, a combination, a medical device, and the like, that use an extract from *Mycobacterium tuberculosis* and are a new modality for treating, preventing, or preventing the recurrence of a cancer or a tumor. The present disclosure provides a composition, a combination, and a medical device, that use an extract from *Mycobacterium tuberculosis* and are a new modality for treating, preventing, or preventing the recurrence of a cancer or a tumor, each of which comprises an immune checkpoint inhibitor and a dendritic cell direct activator or means.

## Description

### [Technical Field]

The present disclosure relates to novel use of a *Mycobacterium tuberculosis* extract. More specifically, the present disclosure relates to combined use of a *Mycobacterium tuberculosis* extract and an anticancer agent, or use for enhancing the action of an anticancer agent.

Currently, examples of known cancer treatment that is commonly performed include surgical therapy (cancer tissue extraction by surgery), chemotherapy (administration of an anticancer agent comprising a molecularly targeted drug), radiation therapy (irradiation of radiation on cancer tissue), immunotherapy (enhancement of an immune function of a patient), and combinations thereof.

### [Summary of Invention]

### [Solution to Problem]

The present disclosure relates to novel use of a *Mycobacterium tuberculosis* extract. More specifically, the present disclosure relates to combined use of a *Mycobacterium tuberculosis* extract and an anticancer agent, or use for enhancing the action of an anticancer agent. The present disclosure provides a novel modality of composition, combination, and medical device for use in treatment, prevention, or prevention of recurrence of cancer or tumor utilizing a *Mycobacterium tuberculosis* extract, wherein the composition, combination, and medical device comprises an immune checkpoint inhibitor and a direct dendritic cell activating agent or means.

Thus, the present disclosure provides the following as examples.

### [Item 1]

A composition for use in activation of CXCL10 production, comprising an immunoadjuvant.

### [Item 2]

The composition of any of the preceding items, wherein the activation of CXCL10 production is in a cell comprising a bone marrow-derived cell.

### [Item 3]

The composition of any of the preceding items, wherein the activation of CXCL10 production is in an antigen-presenting cell.

### [Item 4]

A composition for use in treating cancer or tumor that is CXCL10 positive, comprising an immunoadjuvant.

### [Item 5]

A composition for use in promoting infiltration of a CXCR3 positive cell into cancer or tumor, comprising an immunoadjuvant.

### [Item 6]

A composition for use in promoting infiltration of a CD8 positive T cell into cancer or tumor, comprising an immunoadjuvant.

### [Item 7]

A pharmaceutical composition comprising an immunoadjuvant, characterized in that the composition is used with an anticancer agent or another therapeutic technique for cancer or tumor.

### [Item 8]

A combination medicament of: an anticancer agent or another therapeutic technique for cancer or tumor; and an immunoadjuvant.

### [Item 9]

The pharmaceutical composition or combination medicament of any of the preceding items for use in treating a patient for whom existing therapy was not effective.

### [Item 10]

The pharmaceutical composition or combination medicament of any of the preceding items for use in treatment or prevention of Tumor Mutational Burden^{high} and CD8⁺ T cell^{low} inoperable or metastatic solid cancer for which there is no other therapeutic choice, the pharmaceutical composition or combination medicament comprising an immunoadjuvant.

### [Item 11]

The composition, pharmaceutical composition, or combination medicament of any of the preceding items, wherein the immunoadjuvant comprises a *Mycobacterium tuberculosis* extract or a portion thereof.

### [Item 12]

The composition, pharmaceutical composition, or combination medicament of any of the preceding items, wherein the immunoadjuvant comprises a human *Mycobacterium tuberculosis* hot water extract.

### [Item A1]

A method for activating CXCL10 production in a subject, comprising administering an effective amount of an immunoadjuvant to the subject.

### [Item A2]

The method of any of the preceding items, wherein the activation of CXCL10 production is in a cell comprising a bone marrow-derived cell.

### [Item A3]

The method of any of the preceding items, wherein the activation of CXCL10 production is in an antigen-presenting cell.

### [Item A4]

A method for treating cancer or tumor that is CXCL10 positive in a subject, comprising administering an effective amount of an immunoadjuvant to the subject.

### [Item A5]

A method for promoting infiltration of a CXCR3 positive cell into cancer or tumor in a subject, comprising administering an effective amount of an immunoadjuvant to the subject.

### [Item A6]

A method for promoting infiltration of a CD8 positive T cell into cancer or tumor in a subject, comprising administering an effective amount of an immunoadjuvant to the subject.

### [Item A7]

A method for preventing or treating cancer or tumor in a subject, the method being administering an effective amount of an immunoadjuvant to the subject, wherein administration of the immunoadjuvant is performed in combination with an effective amount of an anticancer agent or another therapeutic technique for cancer or tumor.

### [Item A8]

A method for preventing or treating cancer or tumor in a subject, comprising performing a combination of an effective amount of an immunoadjuvant and an effective amount of an anticancer agent or another therapeutic technique for cancer or tumor.

### [Item A9]

The method of any of the preceding items, wherein the subject is a subject for whom existing therapy was not effective.

### [Item A10]

The method of any of the preceding items, wherein the subject has Tumor Mutational Burden^{high} and CD8⁺ T cell^{low} inoperable or metastatic solid cancer for which there is no other therapeutic choice.

### [Item A11]

The method of any of the preceding items, wherein the immunoadjuvant comprises a *Mycobacterium tuberculosis* extract or a portion thereof.

### [Item A12]

The method of any of the preceding items, wherein the immunoadjuvant comprises a human *Mycobacterium tuberculosis* hot water extract.

### [Item B1]

Use of an immunoadjuvant in the manufacture of a medicament for activation of CXCL10 production.

### [Item B2]

The use of any of the preceding items, wherein the activation of CXCL10 production is in a cell comprising a bone marrow-derived cell.

### [Item B3]

The use of any of the preceding items, wherein the activation of CXCL10 production is in an antigen-presenting cell.

### [Item B4]

Use of an immunoadjuvant in the manufacture of a medicament for treating cancer or tumor that is CXCL10 positive.

### [Item B5]

Use of an immunoadjuvant in the manufacture of a medicament for promoting infiltration of a CXCR3 positive cell into cancer or tumor.

### [Item B6]

Use of an immunoadjuvant in the manufacture of a medicament for promoting infiltration of a CD8 positive T cell into cancer or tumor.

### [Item B7]

Use of an immunoadjuvant in the manufacture of a medicament which is used with an anticancer agent or another therapeutic technique for cancer or tumor.

### [Item B8]

Use of an immunoadjuvant in the manufacture of a medicament which is used in combination with an anticancer agent or another therapeutic technique for cancer or tumor.

### [Item B8A]

The use of any of the preceding items, wherein the medicament is a medicament for prevention or treatment of cancer or tumor.

### [Item B9]

The use of any of the preceding items in the manufacture of a medicament for treating a patient for whom existing therapy was not effective.

### [Item B10]

The use of any of the preceding items in the manufacture of a medicament for treatment or prevention of Tumor Mutational Burden^{high} and CD8⁺ T cell^{low} inoperable or metastatic solid cancer for which there is no other therapeutic choice.

### [Item B11]

The use of any of the preceding items, wherein the immunoadjuvant comprises a *Mycobacterium tuberculosis* extract or a portion thereof.

### [Item B12]

The use of any of the preceding items, wherein the immunoadjuvant comprises a human *Mycobacterium tuberculosis* hot water extract.

### [Item C1]

An immunoadjuvant for use in activation of CXCL10 production.

### [Item C2]

The immunoadjuvant of any of the preceding items, wherein the activation of CXCL10 production is in a cell comprising a bone marrow-derived cell.

### [Item C3]

The immunoadjuvant of any of the preceding items, wherein the activation of CXCL10 production is in an antigen-presenting cell.

### [Item C4]

An immunoadjuvant for use in treating cancer or tumor that is CXCL10 positive.

### [Item C5]

An immunoadjuvant for use in promoting infiltration of a CXCR3 positive cell into cancer or tumor.

### [Item C6]

An immunoadjuvant for use in promoting infiltration of a CD8 positive T cell into cancer or tumor.

### [Item C7]

An immunoadjuvant to be used as a medicament, characterized in that the immunoadjuvant is used with an anticancer agent or another therapeutic technique for cancer or tumor.

### [Item C8]

An immunoadjuvant to be used as a medicament, characterized in that the immunoadjuvant is used in combination with an anticancer agent or another therapeutic technique for cancer or tumor.

### [Item C8A]

The immunoadjuvant of any of the preceding items, wherein the medicament is a medicament for prevention or treatment of cancer or tumor.

### [Item C9]

The immunoadjuvant of any of the preceding items, wherein the medicament is a medicament for treating a patient for whom existing therapy was not effective.

### [Item C10]

The immunoadjuvant of any of the preceding items, wherein the medicament is a medicament for treatment or prevention of Tumor Mutational Burden^{high} and CD8⁺ T cell^{low} inoperable or metastatic solid cancer for which there is no other therapeutic choice.

### [Item C11]

The immunoadjuvant of any of the preceding items, comprising a *Mycobacterium tuberculosis* extract or a portion thereof.

### [Item C12]

The immunoadjuvant of any of the preceding items, comprising a human *Mycobacterium tuberculosis* hot water extract.

The present disclosure also provides the following items.

### [Item X1]

A composition for use in activation of CXCL10 production, comprising an immunoadjuvant.

### [Item X2]

The composition of any of the preceding items, wherein the activation of CXCL10 production is in a cell comprising a bone marrow-derived cell.

### [Item X3]

The composition of any of the preceding items, wherein the activation of CXCL10 production is in an antigen-presenting cell.

### [Item X4]

The composition of any of the preceding items, wherein the activation of CXCL10 production is in a lymph node.

### [Item X5]

A composition for use in treating cancer or tumor that is CXCL10 positive, comprising an immunoadjuvant.

### [Item X6]

A composition for use in promoting infiltration of a CXCR3 positive cell into cancer or tumor, comprising an immunoadjuvant.

### [Item X7]

A composition for use in promoting infiltration of a CD8 positive T cell into cancer or tumor, comprising an immunoadjuvant.

### [Item X8]

A composition for use in activating an immune cell in a lymph node, comprising an immunoadjuvant.

### [Item X9]

A composition for use in promoting infiltration of a CD8 positive T cell into a lymph node, comprising an immunoadjuvant.

### [Item X10]

The composition of any of the preceding items, wherein the CD8 positive T cell is an effector memory CD8⁺ T cell.

### [Item X11]

A pharmaceutical composition comprising an immunoadjuvant, characterized in that the composition is used with an anticancer agent or another therapeutic technique for cancer or tumor.

### [Item X12]

A combination medicament of: an anticancer agent or another therapeutic technique for cancer or tumor; and an immunoadjuvant.

### [Item X13]

The pharmaceutical composition or combination medicament of any of the preceding items for use in treating a patient for whom existing therapy was not effective.

### [Item X14]

The pharmaceutical composition or combination medicament of any of the preceding items for use in treatment or prevention of Tumor Mutational Burden^{high} and CD8⁺ T cell^{low} inoperable or metastatic solid cancer for which there is no other therapeutic choice, the pharmaceutical composition or combination medicament comprising an immunoadjuvant.

### [Item X15]

The composition, pharmaceutical composition, or combination medicament of any of the preceding items, wherein the immunoadjuvant comprises a *Mycobacterium tuberculosis* extract or a portion thereof.

### [Item X16]

The composition, pharmaceutical composition, or combination medicament of any of the preceding items, wherein the immunoadjuvant comprises a human *Mycobacterium tuberculosis* hot water extract.

### [Item X17]

The pharmaceutical composition of any of the preceding items or the combination medicament of any of the preceding items for use in treatment or prevention of Tumor Mutational Burden^{high} and CD8⁺ T cell^{low} inoperable or metastatic solid cancer for which there is no other therapeutic choice, the pharmaceutical composition or combination medicament comprising an immunoadjuvant, wherein the immunoadjuvant comprises a *Mycobacterium tuberculosis* extract or a portion thereof.

### [Item X18]

The pharmaceutical composition or combination medicament of any of the preceding items, wherein the immunoadjuvant comprises a human *Mycobacterium tuberculosis* hot water extract.

### [Item XA1]

A method for activating CXCL10 production in a subject, comprising administering an effective amount of an immunoadjuvant to the subject.

### [Item XA2]

The method of any of the preceding items, wherein the activation of CXCL10 production is in a cell comprising a bone marrow-derived cell.

### [Item XA3]

The method of any of the preceding items, wherein the activation of CXCL10 production is in an antigen-presenting cell.

### [Item XA4]

The method of any of the preceding items, wherein the activation of CXCL10 production is in a lymph node.

### [Item XA5]

A method for treating cancer or tumor that is CXCL10 positive in a subject, comprising administering an effective amount of an immunoadjuvant to the subject.

### [Item XA6]

A method for promoting infiltration of a CXCR3 positive cell into cancer or tumor in a subject, comprising administering an effective amount of an immunoadjuvant to the subject.

### [Item XA7]

A method for promoting infiltration of a CD8 positive T cell into cancer or tumor in a subject, comprising administering an effective amount of an immunoadjuvant to the subject.

### [Item XA8]

A method for activating an immune cell in a lymph node in a subject, comprising administering an effective amount of an immunoadjuvant to the subject.

### [Item XA9]

A method for promoting infiltration of a CD8 positive T cell into a lymph node in a subject, comprising administering an effective amount of an immunoadjuvant to the subject.

### [Item XA10]

The method of any of the preceding items, wherein the CD8 positive T cell is an effector memory CD8⁺ T cell.

### [Item XA11]

A method for preventing or treating cancer or tumor in a subject, the method being administering an effective amount of an immunoadjuvant to the subject, wherein administration of the immunoadjuvant is performed in combination with an effective amount of an anticancer agent or another therapeutic technique for cancer or tumor.

### [Item XA12]

A method for preventing or treating cancer or tumor in a subject, comprising performing a combination of an effective amount of an immunoadjuvant and an effective amount of an anticancer agent or another therapeutic technique for cancer or tumor.

### [Item XA13]

The method of any of the preceding items, wherein the subject is a subject for whom existing therapy was not effective.

### [Item XA14]

The method of any of the preceding items, wherein the subject has Tumor Mutational Burden^{high} and CD8⁺ T cell^{low} inoperable or metastatic solid cancer for which there is no other therapeutic choice.

### [Item XA15]

The method of any of the preceding items, wherein the immunoadjuvant comprises a *Mycobacterium tuberculosis* extract or a portion thereof.

### [Item XA16]

The method of any of the preceding items, wherein the immunoadjuvant comprises a human *Mycobacterium tuberculosis* hot water extract.

### [Item XA17]

The method of any of the preceding items for treating or preventing Tumor Mutational Burden^{high} and CD8⁺ T cell^{low} inoperable or metastatic solid cancer for which there is no other therapeutic choice in a subject, comprising administering an effective amount of an immunoadjuvant to the subject, wherein the immunoadjuvant comprises a *Mycobacterium tuberculosis* extract or a portion thereof.

### [Item XA18]

The method of any of the preceding items, wherein the immunoadjuvant comprises a human *Mycobacterium tuberculosis* hot water extract.

### [Item XB1]

Use of an immunoadjuvant in the manufacture of a medicament for activation of CXCL10 production.

### [Item XB2]

The use of any of the preceding items, wherein the activation of CXCL10 production is in a cell comprising a bone marrow-derived cell.

### [Item XB3]

The use of any of the preceding items, wherein the activation of CXCL10 production is in an antigen-presenting cell.

### [Item XB4]

The use of any of the preceding items, wherein the activation of CXCL10 production is in a lymph node.

### [Item XB5]

Use of an immunoadjuvant in the manufacture of a medicament for treating cancer or tumor that is CXCL10 positive.

### [Item XB6]

Use of an immunoadjuvant in the manufacture of a medicament for promoting infiltration of a CXCR3 positive cell into cancer or tumor.

### [Item XB7]

Use of an immunoadjuvant in the manufacture of a medicament for promoting infiltration of a CD8 positive T cell into cancer or tumor.

### [Item XB8]

Use of an immunoadjuvant in the manufacture of a medicament for activating an immune cell in a lymph node.

### [Item XB9]

Use of an immunoadjuvant in the manufacture of a medicament for promoting infiltration of a CD8 positive T cell into a lymph node.

### [Item XB10]

The use of any of the preceding items, wherein the CD8 positive T cell is an effector memory CD8⁺ T cell.

### [Item XB11]

Use of an immunoadjuvant in the manufacture of a medicament which is used with an anticancer agent or another therapeutic technique for cancer or tumor.

### [Item XB12]

Use of an immunoadjuvant in the manufacture of a medicament which is used in combination with an anticancer agent or another therapeutic technique for cancer or tumor.

### [Item XB12A]

The use of any of the preceding items, wherein the medicament is a medicament for prevention or treatment of cancer or tumor.

### [Item XB13]

The use of any of the preceding items in the manufacture of a medicament for treating a patient for whom existing therapy was not effective.

### [Item XB14]

The use of any of the preceding items in the manufacture of a medicament for treatment or prevention of Tumor Mutational Burden^{high} and CD8⁺ T cell^{low} inoperable or metastatic solid cancer for which there is no other therapeutic choice.

### [Item XB15]

The use of any of the preceding items, wherein the immunoadjuvant comprises a *Mycobacterium tuberculosis* extract or a portion thereof.

### [Item XB16]

The use of any of the preceding items, wherein the immunoadjuvant comprises a human *Mycobacterium tuberculosis* hot water extract.

### [Item XB17]

The use of any of the preceding items in the manufacture of a medicament for treating or preventing Tumor Mutational Burden^{high} and CD8⁺ T cell^{low} inoperable or metastatic solid cancer for which there is no other therapeutic choice, wherein the immunoadjuvant comprises a *Mycobacterium tuberculosis* extract or a portion thereof.

### [Item XB18]

The use of any of the preceding items, wherein the immunoadjuvant comprises a human *Mycobacterium tuberculosis* hot water extract.

### [Item XC1]

An immunoadjuvant for use in activation of CXCL10 production.

### [Item XC2]

The immunoadjuvant of any of the preceding items, wherein the activation of CXCL10 production is in a cell comprising a bone marrow-derived cell.

### [Item XC3]

The immunoadjuvant of any of the preceding items, wherein the activation of CXCL10 production is in an antigen-presenting cell.

### [Item XC4]

The immunoadjuvant of any of the preceding items, wherein the activation of CXCL10 production is in a lymph node.

### [Item XC5]

An immunoadjuvant for use in treating cancer or tumor that is CXCL10 positive.

### [Item XC6]

An immunoadjuvant for use in promoting infiltration of a CXCR3 positive cell into cancer or tumor.

### [Item XC7]

An immunoadjuvant for use in promoting infiltration of a CD8 positive T cell into cancer or tumor.

### [Item XC8]

An immunoadjuvant for use in activating an immune cell in a lymph node.

### [Item XC9]

An immunoadjuvant for use in promoting infiltration of a CD8 positive T cell into a lymph node.

### [Item XC10]

The immunoadjuvant of any of the preceding items, wherein the CD8 positive T cell is an effector memory CD8⁺ T cell.

### [Item XC11]

An immunoadjuvant to be used as a medicament, characterized in that the immunoadjuvant is used with an anticancer agent or another therapeutic technique for cancer or tumor.

### [Item XC12]

An immunoadjuvant to be used as a medicament, characterized in that the immunoadjuvant is used in combination with an anticancer agent or another therapeutic technique for cancer or tumor.

### [Item XC12A]

The immunoadjuvant of any of the preceding items, wherein the medicament is a medicament for prevention or treatment of cancer or tumor.

### [Item XC13]

The immunoadjuvant of any of the preceding items, wherein the medicament is a medicament for treating a patient for whom existing therapy was not effective.

### [Item XC14]

The immunoadjuvant of any of the preceding items, wherein the medicament is a medicament for treatment or prevention of Tumor Mutational Burden^{high} and CD8⁺ T cell^{low} inoperable or metastatic solid cancer for which there is no other therapeutic choice.

### [Item XC15]

The immunoadjuvant of any of the preceding items, comprising a *Mycobacterium tuberculosis* extract or a portion thereof.

### [Item XC16]

The immunoadjuvant of any of the preceding items, comprising a human *Mycobacterium tuberculosis* hot water extract.

### [Item XC17]

The immunoadjuvant of any of the preceding items, which is a medicament for treatment or prevention of Tumor Mutational Burden^{high} and CD8⁺ T cell^{low} inoperable or metastatic solid cancer for which there is no other therapeutic choice, wherein the immunoadjuvant comprises a *Mycobacterium tuberculosis* extract or a portion thereof.

### [Item XC18]

The immunoadjuvant of any of the preceding items, wherein the immunoadjuvant comprises a human *Mycobacterium tuberculosis* hot water extract.

The present disclosure is intended so that one or more of the aforementioned features can be provided not only as the explicitly disclosed combinations, but also as other combinations thereof. Additional embodiments and advantages of the present disclosure are recognized by those skilled in the art by reading and understanding the following detailed description as needed.

### [Advantageous Effects]

The present disclosure can provide a method for effectively treating or preventing cancer or tumor by using an immunoadjuvant or the like such as a *Mycobacterium tuberculosis* extract.

### [Brief Description of Drawings]

[Figure 1] Figure **1** is a diagram showing enhancement of CXCL10 production by an immunoadjuvant in Example 2.
[Figure 2] Figure **2** is a diagram showing an increase in CD8⁺ T cells in tumor by administration of Extract Z in Example 4.

### [Description of Embodiments]

The present disclosure is described hereinafter while showing some of the best modes thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

### (Definitions)

The terms used herein are described hereinafter.

As used herein, "CXCL10" is an abbreviation of C-X-C motif chemokine ligand 10 and is also known as IP-10, interferon gamma-induced protein 10, or Small-inducible cytokine B10. CXCL10 is an 8.7 kDa protein that, in humans, is encoded by the CXCL10 gene, is a non-glycosylated protein, and consists of 77 amino acids. CXCL10 is a chemokine that is produced in response to treatment of a monocyte, an endothelial cell, or a fibroblast with IFNγ. IP-10 functions as a chemotaxis inducer cell expressing a G protein-coupled receptor, CXCR3 that has been found mainly in an activated T cell or NK cell. In addition, IP-10 is also known as CXCL10, C7, IFI10, INP10, IP-10, SCYB10, crg-2, gIP-10, mob-1, C-X-C motif chemokine ligand 10, C-X-C motif chemokine 10, or the like. IP-10 is also known by its IDs, NM_001565 (nucleic acid) or NP_001556 (protein).

As used herein, "activation of CXCL10 production" refers to increasing the production (or amount of substance) of CXCL10.

As used herein, "cancer or tumor that is CXCL10 positive" refers to cancer or tumor being CXCL10 positive. Examples of cancer or tumor that is CXCL10 positive include brain tumor, spinal cord tumor, oral cavity cancer/pharyngeal cancer/nasal cancer, pharyngeal cancer, thyroid cancer, lung cancer, breast cancer, mediastinal tumor, mesothelioma, esophageal cancer, gastric cancer, duodenal/small intestine cancer, colon cancer, GIST, liver cancer, biliary tract cancer/gallbladder cancer, pancreatic cancer, renal cancer, urinary tract cancer, bladder cancer, adrenal tumor, prostate cancer, testicular cancer, cervical cancer/uterine body cancer and ovarian cancer, and the like.

As used herein, "CXCR3" has the same meaning as the meaning commonly used in the art, and is one of CXC chemokine receptor families, which are G protein-coupled receptors. In addition to G protein-coupled receptor 9 (GPR9) and CD183, CXCR3 may be referred to as CD182; CKR-L2; CMKAR3; IP10-R; Mig-R; MigR, or the like. Two mutants of CXCR3 are known. While CXCR3-A, one of the mutants, binds to CXCL9 (MIG), CXCL10 (IP-10), and CXCL11 (I-TAC), which are CXC chemokines, CXCR3-B can further bind to CXCL4 in addition to those chemokines. While examples of the nucleic acid ID include NM_001142797, NM_001504, and the like, examples of the protein ID can include NP_001136269 and NP_001495.

As used herein, a "CD8 positive T cell" refers to a T cell that is positive for CD8 expression. As used herein, infiltration of CD8 positive T cells are evaluated in accordance with the cell count counted by confirming a slide using at least three different high magnification fields (40 power objective and 10 power eyepiece at the maximum). The number of cells stained as CD8 positive is recorded, and the case where the number of cells is 5 or less in three fields is interpreted as low infiltration, while the case where the number of cells is more than 5 is interpreted as high infiltration.

As used herein, an "immunoadjuvant" refers to any agent or factor that assists an immune reaction. Whether a certain substance is an immunoadjuvant can be determined by confirming whether the substance does not constitute a specific antigen, followed by studying whether the substance increases and/or boosts the intensity of an immune reaction to a co-administered antigen. Typical examples of an immunoadjuvant may include, but are not limited to, human *Mycobacterium tuberculosis* hot water extracts or a portion thereof, stimulators for a pattern recognition receptor such as Toll-like receptor or RIG-1 and NOD-like receptor (NLR), mineral salts such as alum, alum combined with monophosphoryl lipid (MPL) A of enteric bacteria such as *Escherihia coli, Salmonella minnesota, Salmonella typhimurium* or *Shigella flexneri,* saponins such as, particularly, MPL^{®} (AS04), QS-21, Quil-A, ISCOM, or ISCOMATRIX^{™}, emulsions such as MF59^{™}, Montanide^{®} , ISA 51 and ISA 720, liposomes and liposomal formulations such as AS02 (QS21 + squalene + MPL^{®}), AS15, or AS01, synthesized or specifically prepared microparticles and microcarriers such as outer membrane vesicles (OMVs) derived from bacteria such as *N*. *gonorrheae* or *Chlamydia trachomatis,* or chitosan particles, deopt-forming agents such as Pluronic^{®} block copolymer, specifically modified or prepared peptides such as muramyl dipeptides, aminoalkyl glucosaminide 4-phosphate such as RC529, or protein such as bacterial toxoids or toxin fragments. Although the present disclosure may be described using a human *Mycobacterium tuberculosis* hot water extract or a portion thereof as a typical example, the present disclosure is not limited thereto.

As used herein, a "human *Mycobacterium tuberculosis* hot water extract" is typically a substance produced from a human *Mycobacterium tuberculosis,* which is a mixture including polysaccharides with arabinose, mannose, and glucose as the primary ingredients. While anticancer effects due to human *Mycobacterium tuberculosis* hot water extracts have been studied for a long time, the detailed mechanism of action thereof is not necessarily elucidated. Further, the extract has not been used as a prophylactic drug. The extract can also comprise a trace amount of ingredients such as a protein, peptide, amino acid, nucleic acid, or lipid (glycolipid) when appropriate. Examples of a human *Mycobacterium tuberculosis* hot water extract can include Extract Z described herein or the like. Thus, as a human *Mycobacterium tuberculosis* hot water extract, the technology of the present disclosure can use Extract Z described in detail herein or any formulation manufactured using Extract Z as an active pharmaceutical ingredient.

The following is a representative manufacturing method of human *Mycobacterium tuberculosis* hot water extracts.

Human *Mycobacterium tuberculosis* is cultured for 3 to 7 weeks in a 37°C thermostatic vessel. The film of microbial cells formed on a medium is then filtered out. The moist microbial cells with medium components removed by washing with water are used as the extracted raw material. The microbial cells are floated in a distilled water at an amount that is 15 to 40-fold of the wet weight thereof, and heated for 80 to 180 minutes at 90 to 120°C for extraction. The residue of the microbial cells is removed with a sterilization filter, and the extracted solution is concentrated to 60% or less, and then acetone, trichloroacetate, ammonium sulfate, sulfosalicylic acid, or the like is added thereto so as to arrive at 0.5 to 3% (w/v), and stirred and left standing. The deposited precipitate is then centrifuged and removed, and the supernatant is subjected to running water dialysis. Inner fraction of dialysis fluid is subjected to vacuum concentration to a 1/20 to 1/4 volume, and sodium chloride is added to the concentrate so as to arrive at 0.5 to 1% (w/v). Two to fourfold volume of ethanol is added and left standing, and then the precipitate is removed by centrifugation. After adding an additional 2 to 6-fold volume of ethanol to the supernatant and incubating, a precipitating polysaccharide is obtained by centrifugation or the like. Those skilled in the art can understand that each of the conditions described above can be appropriately changed to obtain the same product.

As used herein, "prophylaxis" or "prevention" is an act of administering an active ingredient in the present disclosure to an individual who has not developed the target disease, intended to for example prevent development of the disease.

As used herein, "treatment" or "therapy" is, for example, an act of administering an active ingredient of the present disclosure to an individual (subject, patient) diagnosed as having a developed disease by a physician or a similar practitioner, intended to, for example, alleviate the disease or condition, not increase carcinoma, or revert back to the state before the development of the disease. Even if the objective of administration is prevention of exacerbation of the disease or condition or prevention of increase in the carcinoma, the administration is a therapeutic act if administered to a patient.

As used herein, "existing therapy" refers to any therapy that is currently available, and refers to, for example, therapy that is recommended for each cancer type and stage according to the guidelines prescribed by an organization such as a cancer association of each country. Examples of cancer therapy can include surgical operation therapy, radiation therapy, chemotherapy, immunotherapy (e.g., immune cell therapy/immune checkpoint inhibitor/photoimmunotherapy or the like), molecularly targeted drugs, and the like.

Examples of the guidelines prescribed by an organization such as a cancer association of each country include the guidelines (https://www.nccn.org/guidelines/category_1) provided by the US National Comprehensive Cancer Network (NCCN), and the like. Further, examples of the guidelines provided by an association in Japan include the following guidelines. Note that it is understood that these guidelines are new editions updated in accordance with accumulation of scientific finding.

**[Table A]**

| **Carcinoma** | **Association/organi zation** | **Guidelines** | |
|---|---|---|---|
| Brain tumor | The Japan Society for Neuro-Oncology | Practical Guidelines for Neuro-Oncology/Adu lt Brain Tumor Part | 2019 edition |
| Head and neck cancer | Japan Society for Head and Neck Cancer | Japanese Clinical Practice Guidelines for Head and Neck Cancer | 2018 edition |
| Oral cavity cancer | Japanese Society for Oral Oncology/Japanese Society of Oral and Maxillofacial Surgeons | Clinical Practice Guidelines for Oral Cancer | 2019 edition |
| Thyroid cancer | Japan Association of Endocrine Surgery/Japanese Society of Thyroid Surgery | Guidelines for Diagnosis and Treatment of Thyroid Cancer | 2018 edition |
| Lung cancer | The Japan Lung Cancer Society | Guidelines for Diagnosis and Treatment of Lung Cancer | 2020 edition ver1.1 |
| Breast cancer | Japanese Breast Cancer Society | Japanese Breast Cancer Society Clinical Practice Guidelines for Breast Cancer | 2018 edition [supplem ent 2019] |
| Esophageal cancer | The Japan Esophageal Society | Guidelines for Diagnosis and Treatment of Carcinoma of the Esophagus | 2017 edition |
| Gastric cancer | Japanese Gastric Cancer Association | Guideline for Treatment of Gastric Cancer | For Physicia ns, revised in July 2021, the 6th edition |
| Duodenal cancer | Japan Duodenal Cancer Guideline Committee | Clinical Practice Guidelines for Duodenal Cancer | 2021 edition |
| Colon cancer | Japanese Society for Cancer of the Colon and Rectum | JSCCR Guidelines for the Treatment of Colorectal Cancer | For physicia ns, 2019 edition |
| GIST | Japan Society of Clinical Oncology, GIST Guideline Committee | Japanese Clinical Practice Guidelines for Gastrointest inal Stromal Tumors (GIST) | The 3rd edition, 2014 |
| Liver cancer | The Japan Society of Hepatology | JSH HCC Guidelines | 2017 edition, revised edition |
| Bile duct cancer | Japanese Society of Hepato-Biliary-Pancreatic Surgery | Guidelines for Treatment of Bile Duct Cancer | The 3rd edition |
| Pancreatic cancer | Japan Pancreas Society | Clinical Practice Guidelines for Pancreatic Cancer | 2019 |
| Renal cancer | The Japanese Urological Association | Guidelines for Treatment of Renal Cancer | 2017 edition |
| Renal pelvis/ureter al cancer | The Japanese Urological Association | Guidelines for Treatment of Renal Pelvis/Urete ral Cancer | 2014 edition |
| Bladder cancer | The Japanese Urological Association | Guidelines for Treatment of Bladder Cancer | 2019 edition |
| Prostate cancer | The Japanese Urological Association | Clinical Practice Guidelines for Prostate Cancer | 2016 edition |
| Testicular cancer | The Japanese Urological Association | Guidelines for Treatment of Testicular Cancer | 2015 edition |
| Cervical cancer | Japan Society of Gynecologic Oncology | Guidelines for treatment of uterine cervical cancer | 2017 edition |
| Uterine body cancer | Japan Society of Gynecologic Oncology | Guidelines for treatment of uterine body neoplasm | 2018 edition |
| Ovarian cancer/fallop ian tube cancer/perito neal cancer | Japan Society of Gynecologic Oncology | Guidelines for treatment of ovarian cancer, fallopian tube cancer and primary peritoneal cancer | 2020 edition |

As used herein, "there is no other therapeutic choice" refers to a condition in which performing therapy that is recommended for each cancer type and stage according to the guidelines prescribed by an organization such as a cancer association of each country did not result in cure, or a health condition in which such therapy cannot be performed. The "therapy that is recommended for each cancer type and stage according to the guidelines prescribed by an organization such as a cancer association of each country" is based on the guidelines described in the "existing therapy" herein.

As used herein, "Tumor Mutational Burden^{high} and CD8⁺ T cell^{low} inoperable or metastatic solid cancer" refers to cancer or tumor having a greater amount of gene mutation as compared to a reference genome as a result of reading the DNA of a cancer cell and having a less CD8⁺ T cell amount or proportion in a peripheral blood mononuclear cell or tumor tissue as a result of measurement using an approach such as flow cytometry or immunostaining, wherein surgical operation therapy is not applied to the cancer or tumor, or the cancer or tumor has metastasized.

As used herein, the term "co-administer" or "co-administration" refers to administration of two or more types of agents within a certain period from each other (e.g., a period of 24 hours) as, for example, a part of a clinical treatment regimen. In another embodiment, "co-administer" or "co-administration" refers to administration of two or more types of agents within 2 hours from each other. In another embodiment, "co-administer" or "co-administration" refers to administration of two or more types of agents within 30 minutes from each other. In another embodiment, "co-administer" or "co-administration" refers to administration of two or more types of agents within 15 minutes from each other. In another embodiment, "co-administer" or "co-administration" refers administration of agents at the same time either as a part of a single formulation or as a plurality of formulations that are administered via the same route or different routes.

As used herein, a "subject" refers to an animal including warm-blooded mammalians such as primates including humans; birds; rearing animals or domestic animals such as cats, dogs, sheep, goats, cows, horses, or pigs; experimental animals such as mice, rats, or guinea pigs; fish; reptiles; rearing animals and wild animals, and the like, and is preferably primates, and more preferably includes humans.

As used herein, a "bone marrow-derived cell" refers to any cell derived from the bone marrow, and includes, but is not limited to, hematopoietic stem cells and white blood cells, red blood cells and platelets derived therefrom, differentiated cells such as osteoblasts or fibrocytes, or stem cells represented by cells that have been called bone marrow mesenchymal stem cell, bone marrow interstitial pluripotent stem cell, or bone marrow pluripotent stem cell. A "bone marrow-derived cell" can be isolated from a subject by an approach such as bone marrow (cell) collection or peripheral blood collection. The present invention also encompasses a method of isolating a "bone marrow-derived cell" from a subject and treating the isolated cell with an agent, followed by returning the cell into the body of the subject, thereby performing therapy.

As used herein, an "antigen-presenting cell" refers to a heterogeneous group of immune responsive cells that mediate a cell-mediated immune response by processing and presenting an antigen to a T cell. Examples of an antigen-presenting cell include, but are not limited to, macrophages, dendritic cells, Langerhans cells, B-lymphocytes, platelets, and artificial antigen-presenting cells (aAPCs).

As used herein, an "anticancer agent or another therapeutic technique for cancer or tumor" may be also referred to as an "anticancer agent or the like", and refers to a substance or a factor or a means responsible for any cancer therapeutic technique such as radiation therapy as well as an anticancer agent that is any substance or factor or means having some type of action on cancer or tumor. When an "anticancer agent" is used herein, it is broadly understood that the anticancer agent includes an "anticancer agent or another therapeutic technique for cancer or tumor".

Examples of an anticancer agent or the like can include a large number of anticancer agents such as agents that induce apoptosis; polynucleotides (e.g., antisense, ribozyme, or siRNA); polypeptides (e.g., enzyme and antibody); biological mimetics; alkaloids; alkylating agents; antitumor antibiotics; metabolic antagonists; hormones; platinum compounds; monoclonal or polyclonal antibodies (e.g., anticancer agent, toxin, antibody binding to defensin) or toxins; radionuclides; biological reaction modifiers (e.g., interferon (e.g., IFN-α) and interleukin (e.g., IL-2)); adoptive immunotherapeutic agents; hematopoietic growth factors; agents that induce differentiation of tumor cells (e.g., all-trans-retinoic acid); gene therapeutic reagents (e.g., antisense therapeutic reagent and nucleotide); tumor vaccines; angiogenesis inhibitors; proteasome inhibitors: NF-KB modulators; anti-CDK compounds; or HDAC inhibitors. An anticancer agent or the like includes a mediator that induces or stimulates apoptosis. Examples of a mediator that induces apoptosis include, but are not limited to, radiation (e.g., X-ray, gamma ray, or UV); tumor necrosis factor (TNF)-associated factors (e.g., TNF family receptor protein, TNF family ligand, or antibody against TRAIL, TRAIL-R1 or TRAIL-R2); kinase inhibitors (e.g., epidermal growth factor receptor (EGFR) kinase inhibitor, vascular growth factor receptor (VGFR) kinase inhibitor, fibroblast growth factor receptor (FGFR) kinase inhibitor, platelet-derived growth factor receptor (PDGFR) kinase inhibitor, and Bcr-Abl kinase inhibitor (such as GLEEVEC)); antisense molecules; antibodies (e.g., herceptin, rituxan, zevalin, and avastin); anti-estrogens (e.g., raloxifene and tamoxifen); anti-androgens (e.g., flutamide, bicalutamide, finasteride, aminoglutethamide, ketoconazole, and corticosteroid); cyclooxygenase 2 (COX-2) inhibitors (e.g., celecoxib, meloxicam, NS-398, and non-steroidal anti-inflammatory drug (NSAID)); anti-inflammatory drugs (e.g., butazolidin, decadron, deltasone, dexamethasone, dexamethasone intensol, dexon, hexadrol, hydroxychlorquine, meticorten, oradexon, orasone, oxyphenbutazone, PEDIAPRED, phenylbutazone, PLAQUENIL, prednisolone, prednisone, prelone, and tanderil); and chemotherapeutic drugs for cancer (e.g., irinotecan (CAMPTOSAR), CPT-11, fludarabine (FLUDARA), dacarbazine (DTIC), dexamethasone, mitoxantrone, mylotarg, VP-16, cisplatin, carboplatin, oxaliplatin, 5-FU, doxorubicin, gemcitabine, bortezomib, gefitinib, bevacizumab, taxotere or taxol); cellular signaling molecules; ceramides and cytokines; staurosporines; immune checkpoint inhibitors and the like.

As used herein, an "immune checkpoint inhibitor" is a substance capable of disabling suppression of activation of a T cell by an immune checkpoint molecule by binding to an immune checkpoint molecule or a ligand thereof to inhibit immunosuppressive signaling. Specific examples include inhibitors against PD-1, PD-L1, PD-L2, CTLA-4, LAG-3, TIM-1, TIM-3, TIM-4, VISTA, BTLA, TIGIT, A2AR, 4-1BB, 4-1BBL, 2B4 (CD244), KIR family receptors, B7.1, B7.2, B7-H2, B7-H3, B7-H4, B7-H6, BATE, CD39, CD40, CD47, CD48, CD73, CD94/NKG2A, CD96, CD160, CD200, CD200R, CD274, butyrophilins, CEACAM1, CSF-1R, DcR3, EDO, Foxp1, GARP, GITR, gp49B, HHLA2, HVEM, ICOS, IDO, ILT-2, ILT-4, LAIR-1, MAFB, MICA./B, NKG2A/HLA-E, NR4A2, OCT-2, OX-40, PIR-B, Rara (retinoic acid receptor alpha), SIRP, TDO, TLR3, and TNFR. Preferred examples include inhibitors against PD-1, PD-L1, and CTLA-4. An anti-PD-1 antibody, which is one of PD-1 inhibitors, binds to PD-1 on a T cell to inhibit binding between PD-1 and PD-L1, thereby blocking suppressive signaling and maintaining activation of the T cell. An anti-PD-L1 antibody, which is one of PD-L1 inhibitors, binds to PD-L1 expressed on a cancer cell or an antigen-presenting cell to inhibit interaction with PD-1 on a T cell. As a result, suppressive signaling to the T cell is inhibited, and activation of the T cell is maintained. An anti-CTLA-4 antibody, which is one of CTLA-4 inhibitors, competes with a CD28 ligand on a dendritic cell and blocks a suppressive signal of an immune cell via CD28 to maintain activation of a T cell. Examples of an anti-PD-1 antibody include nivolumab, pembrolizumab, spartalizumab, and cemiplimab. Examples of an anti-PD-L1 antibody include atezolizumab, durvalumab, and avelumab. Examples of an anti-CTLA-4 antibody include ipilimumab and tremelimumab.

Further, in another embodiment, the composition and the method of the present disclosure provide at least one anti-hyper-proliferative agent or antitumor drug selected from the compound of the present disclosure and an alkylating agent, a metabolic antagonist, and a natural product (e.g., herb and other plants and/or animal-derived compound).

Examples of an alkylating agent that is suitable for use in the present composition and method include, but are not limited to, the following: 1) nitrogen mustard (e.g., mechlorethamine, cyclophosphamide, ifosfamide, melphalan (L-sarcolysin); and chlorambucil); 2) ethyleneimine and methylmelamine (e.g., hexamethylmelamine and thiotepa); 3) alkyl sulfonate (e.g., busulfan); 4) nitrosourea (e.g., carmustine (BCNU); lomustin (CCNU); semustine (methyl CCNU); and streptozocin (streptozotocin)); and 5) triazene (e.g., dacarbazine (DTIC; dimethyl-triazeno-imidazole-carboxamide).

In some embodiments, examples of a metabolic antagonist that is suitable for use in the present composition and method include, but are not limited to, the following: 1) folic acid analogs (e.g., methotrexate (amethopterin)); 2) pyrimidine analogs (e.g., fluorouracil (5-fluorouracil; 5-FU), floxuridine (fluorodeoxyuridine; FudR), and cytarabine (cytosine arabinoside)); and 3) purine analogs (e.g., mercaptopurine (6-mercaptopurine; 6-MP), thioguanine (6-thioguanine; TG), and pentostatin (2'-deoxycoformycin)).

Further, in another embodiment, examples of a chemotherapeutic agent that is suitable for use in the present composition and method include, but are not limited to, the following: 1) vinca alkaloid (e.g., vinblastine (VLB) or vincristine); 2) epipodophyllotoxin (e.g., etoposide and teniposide); 3) antibiotics (e.g., dactinomycin (actinomycin D), daunorubicin (daunomycin; rubidomycin), doxorubicin, bleomycin, plicamycin (mithramycin), and mitomycin (mitomycin C)); 4) enzymes (e.g., L-asparaginase); 5) biological reaction modifiers (e.g., interferon alpha); 6) platinum coordination complexes (e.g., cisplatin (cis-DDP) and carboplatin); 7) anthracenedione (e.g., mitoxantrone); 8) substituted urea (e.g., hydroxyurea); 9) methylhydrazine derivatives (e.g., procarbazine (N-methylhydrazine; MIH)); 10) adrenocortical inhibitors (e.g., mitotane (o,p'-DDD) and aminoglutethimide); 11) adrenocortical steroids (e.g., prednisone); 12) progestin (e.g., hydroxyprogesterone caproate, medroxyprogesterone acetate, and megestrol acetate); 13) estrogens (e.g., diethylstilbestrol and ethynyl estradiol); 14) anti-estrogens (e.g., tamoxifen); 15) androgens (e.g., testosterone propionate and fluoxymesterone); 16) anti-androgens (e.g., flutamide); and 17) gonadotropin-releasing hormone analogs (e.g., leuprolide).

Examples of an anticancer agent can include 5-fluorouracil, afatinib, aprindine, azaribine, anastrozole, anthracycline, axitinib, AVL-101, AVL-291, bendamustine, bleomycin, bortezomib, bosutinib, bryostatin-1, busulfan, calicheamicin, camptothecin, carboplatin, 10-hydroxycamptothecin, carmustine, celecoxib, chlorambucil, cisplatinum, COX-2 inhibitor, irinotecan (CPT-11), SN-38, carboplatin, cladribine, camptothecin, crizotinib, cyclophosphamide, cytarabine, dacarbazine, dasatinib, dinaciclib, docetaxel, dactinomycin, daunorubicin, DM1, DM3, DM4, doxorubicin, 2-pyrrolinodoxorubicin (2-PDox), prodrug forms of 2-PDox (pro-2-PDox), cyano-morpholino doxorubicin, doxorubicin glucuronide, endostatin, epirubicin glucuronide, erlotinib, estramustine, epipodophyllotoxin, erlotinib, entinostat, estrogen receptor binding agents, etoposide (VP16), etoposide glucuronide, etoposide phosphate, exemestane, fingolimod, floxuridine (FUdR), 3',5'-O-dioleoyl-FudR (FUdR-dO), fludarabine, flutamide, farnesyl-protein transferase inhibitors, flavopiridol, fostamatinib, ganetespib, GDC-0834, GS-1101, gefitinib, gemcitabine, hydroxyurea, ibrutinib, idarubicin, idelalisib, ifosfamide, imatinib, lapatinib, lenalidomide, leucovorin, LFM-A13, lomustine, mechlorethamine, melphalan, mercaptopurine, 6-mercaptopurine, methotrexate, mitoxantrone, mithramycin, mitomycin, mitotane, monomethyl auristatin F (MMAF), monomethyl auristatin D (MMAD), monomethyl auristatin E (MMAE), navelbine, neratinib, nilotinib, nitrosourea, olaparib, peliomycin, procarbazine, paclitaxel, PCI-32765, pentostatin, PSI-341, raloxifene, semustine, SN-38, sorafenib, streptozocin, SU11248, sunitinib, tamoxifen, temozolomide, transplatin, thalidomide, thioguanine, thiotepa, teniposide, topotecan, uracil mustard, vatalanib, vinorelbine, vinblastine, vincristine, vinca alkaloid and ZD1839, and pharmaceutically acceptable salts thereof.

The term "prodrug" as used herein refers to a pharmacologically inactive derivative of a parent "drug" molecule that requires a (e.g., natural or enzymatic) change in the living body in the physiological system of a target for releasing the prodrug or (e.g., enzymatically, physiologically, mechanically, or electromagnetically) converting the prodrug into an active drug. A prodrug is designed to overcome a problem associated with stability, water solubility, toxicity, lack of specificity, or limited bioavailability efficiency. An exemplary prodrug comprises an active drug molecule itself and a chemical masking group (e.g., a group which reversibly suppresses the activity of the drug). Some prodrugs are variations or derivatives of a compound having a group that is cleavable under a metabolic condition. A prodrug can be readily prepared from the parent compound using the methods known in the art, e.g., the methods described in A Textbook of Drug Design and Development, Krogsgaard-Larsen and H.Bundgaard (eds.), Gordon & Breach, 1991, particularly Chapter 5: "Design and Applications of Prodrugs"; Design of Prodrugs, H.Bundgaard (ed.), Elsevier, 1985; Prodrugs: Topical and Ocular Drug Delivery, K.B.Sloan (ed.), Marcel Dekker, 1998; Methods in Enzymology, K.Widder et al. (eds.), Vol.42, Academic Press, 1985, particularly pp.309-396; Burger's Medicinal Chemistry and Drug Discovery, 5th Ed., M.Wolff (ed.), John Wiley & Sons, 1995, particularly Vol.1 pp.172-178 and pp.949-982; Pro-Drugs as Novel Delivery Systems, T.Higuchi and V.Stella (eds.), Am.Chem.Soc., 1975; and Bioreversible Carriers in Drug Design, E.B.Roche (ed.), Elsevier, 1987.

An exemplary prodrug becomes pharmaceutically active *in vivo* or *in vitro* when solvolysis is performed under a physiological condition or when an enzymatic degradation or other biochemical transformations (e.g., phosphorylation, hydrogenation, dehydrogenation, or glycosylation) is performed. A prodrug often offers advantages in water solubility, histocompatibility, or delayed release in mammalian organisms (for example, see Bundgard, Design of Prodrugs, pp.7-9, 21-24, Elsevier, Amsterdam (1985); and Silverman, The Organic Chemistry of Drug Design and Drug Action, pp.352-401, Academic Press, San Diego, CA (1992)). Examples of a general prodrug include acid derivatives, e.g., esters prepared by a reaction of a parent acid with a suitable alcohol (e.g., lower alkanol) or esters prepared by a reaction of a parent alcohol with a suitable carboxylic acid (e.g., amino acid), amides prepared by a reaction of the parent acid compound with an amine, basic groups reacted to form an acylated base derivative (e.g., lower alkyl amide), or phosphorous-containing derivatives, e.g., phosphoric acid including cyclic phosphoric acid, phosphonic acid, and phosphoramidate, phosphonic acid, and phosphoramidateester (for example, see US Patent Application Publication No. 2007/0249564A1, whose entire content is incorporated herein by reference).

The term "pharmaceutically acceptable salt" as used herein refers to any salt (e.g., a salt obtained by a reaction with an acid or a base) of the compound of the present disclosure which is physiologically accepted in the target animal (e.g., a mammal). A salt of the compound of the present disclosure can be obtained from an inorganic or organic acid and a base. Examples of an acid include, but are not limited to, hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, ethanesulfonic acid, formic acid, benzoic acid, malonic acid, sulfonic acid, naphthalene-2-sulfonic acid, benzenesulfonic acid, and the like. Although other acids such as oxalic acid themselves are not pharmaceutically accepted, such acids may be used for preparation of a salt that is useful as an intermediate in obtaining the compound of the present disclosure and a pharmaceutically acceptable acid addition salt thereof.

Examples of a base include, but are not limited to, alkali metal (e.g., sodium) hydroxides, alkaline earth metal (e.g., magnesium) hydroxides, ammonium, and compounds of formula NW₄⁺ (wherein W is C₁₋₄ alkyl) and the like.

Examples of a salt include, but are not limited to, acetic acid salt, adipic acid salt, alginic acid salt, aspartic acid salt, benzoic acid salt, benzenesulfonic acid salt, bisulfuric acid salt, butyric acid salt, citric acid salt, camphoric acid salt, camphorsulfonic acid salt, cyclopentanepropionic acid salt, digluconic acid salt, dodecylsulfuric acid salt, ethanesulfonic acid salt, fumaric acid salt, flucoheptanoic acid salt (flucoheptanoate), glycerophosphoric acid salt, hemisulfuric acid salt, heptanoic acid salt, hexanoic acid salt, chloride, bromode, iodide, 2-hydroxy ethane sulfonic acid salt, lactic acid salt, maleic acid salt, mesylic acid salt, methanesulfonic acid salt, 2-naphthalenesulfonic acid salt, nicotinic acid salt, oxalic acid salt, palmoate, pectinic acid salt, persulfuric acid salt, phenylpropionic acid salt, picric acid salt, pivalic acid salt, propionic acid salt, succinic acid salt, tartaric acid salt, thiocyanic acid salt, tosylic acid salt, undecanoic acid salt, and the like. Other examples of a salt include an anion of the compound of the present disclosure having a suitable cation such as Na⁺, NH₄⁺, and NW₄⁺ (wherein W is a C₁₋₄ alkyl group). For therapeutic use, a salt of the compound of the present disclosure is contemplated as being pharmaceutically acceptable. However, for example, use in preparation or purification of a pharmaceutically acceptable compound may also be found for a salt of an acid and a base which is not pharmaceutically acceptable.

The term "solvate" as used herein refers to physical association of the compound of the present disclosure and one or more solvent molecules, regardless of whether the solvent molecules are organic or inorganic. This physical association often includes hydrogen bond. In some cases, the solvate can be isolated when, for example, one or more solvate molecules are incorporated in the crystal lattice of the crystalline solid. A "solvate" encompasses both a solution phase solvate and an isolatable solvate. Examples of a typical solvate include hydrates, ethanolates, and methanolates.

A "therapeutically effective amount" refers to an amount of a compound to be administered which prevents a state or alleviates one or more disorder symptoms being treated to some extent. A pharmaceutical composition that is suitable for use herein includes a composition comprising an active ingredient at a sufficient amount for achieving the intended objective. Particularly, given the detailed disclosure provided herein, determination of a therapeutically effective amount sufficiently remains within the scope of the ability of those skilled in the art. As used herein, treatment refers to inhibition of, reduction in, elimination of, or mitigation of, and prevention of a disease.

A pharmaceutical composition can be prepared by any suitable method such as well-known methods in the pharmaceutical field, e.g., the methods described in Gennaro et al., Remington's Pharmaceutical Sciences (the 18th edition, Mack Publishing Co., 1990), particularly Part 8: Pharmaceutical Preparations and their Manufacture, and the like. Such methods comprise a step of combining a compound with a carrier or a diluent and optionally one or more types of supplemental components. Such supplemental components include those that are common in the art, e.g., fillers, binding agents, excipients, disintegrants, lubricants, colorants, flavoring agents, sweeteners, preservatives (e.g., anti-microbial preservatives), suspending agents, thickners, emulsifiers, and/or humectants.

The term "carrier" as used herein refers to a pharmaceutically acceptable substance, composition, or excipient such as, for example, a liquid or solid bulking agent, diluent, additive, solvent, or capsule forming agent, which is associated with or enables the transport or carriage of a target pharmaceutical compound from an organ or portion of the body to another organ or portion of the body. "Pharmaceutically acceptable" refers to being compatible with other raw materials in a formulation and being harmless to patients. Non-limiting examples of pharmaceutically acceptable carriers, carriers, and/or diluents can include sugars such as lactose, glucose, and sucrose, starch such as corn starch and potato starch, cellulose and derivatives thereof such as carboxymethylcellulose sodium, ethyl cellulose, and cellulose acetate, excipients such as powdered tragacanth, malt, gelatin, talc, cocoa powder, and suppository wax, oil such as peanut oil, cotton seed oil, safflower oil, sesame oil, olive oil, corn oil, and soybean oil, glycols such as propylene glycol, polyols such as glycerin, sorbitol, mannitol, and polyethylene glycol, esters such as ethyl oleate and ethyl laureate, buffering agents such as agar, magnesium hydroxide, and aluminum hydroxide, alginic acid, pyogenic substance-free water, isotonic saline, Ringer's solution, ethyl alcohol, phosphate buffer, and other nontoxic compatible substances used in a pharmaceutical formulation. A humectant, emulsifier, and lubricant such as sodium lauryl sulfate, magnesium stearate, or polyethylene oxide-polypropylene oxide copolymer, as well as a colorant, releasing agent, coating agent, sweetener, flavoring agent, fragrance, preservative, and antioxidant can also be included in a composition.

As used herein, "parenteral administration" refers to a dosage form for any route that is not oral administration. Any mode for administration in a mode and level that are effective for treating or preventing a disease intended for cancer treatment or prevention is employed. Examples of means of parenteral administration include administration through transdermal absorption or transmucosal absorption, as well as injection, infusion, and combinations thereof. For example, administration through transdermal absorption or transmucosal absorption exerts an effect by contacting a transdermally absorbed formulation such as a paste agent, adhesive formulation, or spray with the skin or mucous membrane so that a drug in the formulation migrates into the body through the skin or mucous membrane. Examples of administration via injection or infusion include intravenous, intradermal, subcutaneous, intramuscular, and enteral administration (intestinal infusion), which can also be administered as a bolus and/or sustained infusion. Injection or infusion can use a suspension, liquid agent, emulsion, or implanted agent in an oily or aqueous medium, comprising another formulation substance such as a suspending agent, stabilizer, and/or a dispersant. Enteral administration (intestinal infusion) can provide sustained drug delivery to the proximal small intestine by using a tube and a portable infusion pump by percutaneous endoscopic gastrostomy. More preferably, the administration can be subcutaneous or intradermal administration. Parenteral administration (e.g., transdermal administration) can be performed with a tape/patch agent or powder, spray, ointment, paste, cream, lotion, gel, solution, or the like. A composition suitable for parenteral administration can comprise at least one type of a pharmaceutically acceptable aseptic isotonic aqueous or non-aqueous solution, dispersant, suspension, emulsion, implanted agent, or aseptic powder that can be reconstituted in an aseptic injection solution or dispersant immediately before use.

As used herein, a "radiation therapy providing means" refers to a method, an apparatus, an equipment, an agent, a device and the like for providing radiation therapy to a subject.

As used herein, a "medical device" refers to an object that is inserted or transplanted into a target or applied to the surface of a target for treatment, prevention, or prevention of recurrence. In addition to any device that is used for radiation therapy, general examples of the medical device include stents, fasteners, ports, catheters, scaffolds, grafts, and the like.

### (Description of preferred embodiments)

The preferred embodiments of the present disclosure are described hereinafter. It is understood that the embodiments provided hereinafter are provided to better facilitate the understanding of the present disclosure, and thus the scope of the present disclosure should not be limited by the following descriptions. Thus, it is apparent that those skilled in the art can refer to the descriptions herein to make appropriate modifications within the scope of the present disclosure. It is also understood that the following embodiments of the present disclosure can be used alone or in combination.

### (Activation of CXCL10 production)

In one aspect, the present disclosure provides a composition for use in activation of CXCL10 production, comprising an immunoadjuvant (e.g., a *Mycobacterium tuberculosis* extract-related substance such as a *Mycobacterium tuberculosis* hot water extract), use thereof, a method of prevention or treatment using the same, and an immunoadjuvant for use thereof. It is not conventionally expected that an immunoadjuvant represented by a *Mycobacterium tuberculosis* extract-related substance such as a *Mycobacterium tuberculosis* hot water extract can activate CXCL10 production, which was discovered for the first time in the present disclosure and is considered as being significant.

In one embodiment, activation of CXCL10 production is in a cell comprising a bone marrow-derived cell. Being capable of activating CXCL10 production in a cell comprising a bone marrow-derived cell plays an important role in chemical induction for a monocyte, a macrophage, a T cell, an NK cell, or a dendritic cell, bonding of a T cell to an endothelial cell, anticancer activity, spinal cord colony formation, angiogenesis or the like, plays an important role in immunotherapy, and is also expected to exert a significant effect in treatment of a disease such as cancer, tumor, or infection.

In one embodiment, activation of CXCL10 production is in an antigen-presenting cell. CXCL10 production being activated in an antigen-presenting cell plays an important role in immunotherapy and is also expected to exert a significant effect in treatment of a disease such as cancer, tumor, or infection.

In another embodiment, activation of CXCL10 production is in a lymph node. CXCL10 production being activated in a lymph node promotes infiltration of an immune cell into tumor and is also expected to exert a significant effect in treatment of a disease such as cancer, tumor, or infection. Regarding activation of CXCL10 production, since infiltration of an immune cell into tumor is an action via lymph tissue including a lymph node, activation of a lymph node enables a composition comprising an immunoadjuvant (e.g., a *Mycobacterium tuberculosis* extract-related substance such as a *Mycobacterium tuberculosis* hot water extract) to promote infiltration of an immune cell into tumor. In addition, since a lymph node is a tissue that includes an immune cell such as a T cell, a B cell, a macrophage, a dendritic cell, or a plasma cell and executes an immune reaction against an exogenous non-autologous antigen, activation of a lymph node is also expected to exert a significant effect in treatment of a disease such as an infection.

In another aspect, the present disclosure provides a composition for use in treating cancer or tumor that is CXCL10 positive, comprising an immunoadjuvant (e.g., a *Mycobacterium tuberculosis* extract-related substance such as a *Mycobacterium tuberculosis* hot water extract), use thereof, a method of prevention or treatment using the same, and an immunoadjuvant for use thereof. It is not conventionally expected that an immunoadjuvant represented by a *Mycobacterium tuberculosis* extract-related substance such as a *Mycobacterium tuberculosis* hot water extract can be used for treating cancer or tumor that is CXCL10 positive, which was discovered for the first time in the present disclosure and is considered as being significant. It was discovered in the present disclosure that the presence of a cancer or tumor cell that is CXCL10 positive promotes infiltration of a CD8 positive cell into cancer or tumor. This is significant in that a companion medicament using being CXCL10 positive as an indicator can also be provided.

In another aspect, the present disclosure provides a composition for use in promoting infiltration of a CD8 positive T cell into cancer or tumor, comprising an immunoadjuvant (e.g., a *Mycobacterium tuberculosis* extract-related substance such as a *Mycobacterium tuberculosis* hot water extract), the use thereof, a method of prevention or treatment using the same, and an immunoadjuvant for use thereof. Although not wishing to be bound by any theory, the present disclosure found that administration of an immunoadjuvant (e.g., a *Mycobacterium tuberculosis* extract-related substance such as a *Mycobacterium tuberculosis* hot water extract) promotes infiltration of a CD8 positive T cell and improves treatment.

### (Anticancer agent)

The present disclosure provides a pharmaceutical composition, a combination, a method for treatment or prevention of cancer or tumor, use, or use for the manufacture of a medicament, comprising an immunoadjuvant (e.g., a *Mycobacterium tuberculosis* extract-related substance such as a *Mycobacterium tuberculosis* hot water extract) and being characterized in that the pharmaceutical composition, combination, method or use is used with an anticancer agent or another therapeutic technique for cancer or tumor. In this regard, the anticancer agent or another therapeutic technique for cancer or tumor is different from the immunoadjuvant (e.g., a *Mycobacterium tuberculosis* extract-related substance such as a *Mycobacterium tuberculosis* hot water extract).

In another aspect, the present disclosure provides a combination medicament of: an anticancer agent or another therapeutic technique for cancer or tumor; and an immunoadjuvant (e.g., a *Mycobacterium tuberculosis* extract-related substance). The present disclosure provides a pharmaceutical composition, a combination, a method for treatment or prevention of cancer or tumor, use, or use for the manufacture of a medicament. In this regard, the anticancer agent or another therapeutic technique for cancer or tumor is different from the immunoadjuvant (e.g., a *Mycobacterium tuberculosis* extract-related substance such as a *Mycobacterium tuberculosis* hot water extract).

In the present disclosure, examples of an anticancer agent or another therapeutic technique for cancer or tumor that can be used can include a large number of anticancer agents such as agents that induce apoptosis; polynucleotides (e.g., antisense, ribozyme, or siRNA); polypeptides (e.g., enzyme and antibody); biological mimetics; alkaloids; alkylating agents; antitumor antibiotics; metabolic antagonists; hormones; platinum compounds; monoclonal or polyclonal antibodies (e.g., anticancer agent, toxin, antibody binding to defensin) or toxins; radionuclides; biological reaction modifiers (e.g., interferon (e.g., IFN-α) and interleukin (e.g., IL-2)); adoptive immunotherapeutic agents; hematopoietic growth factors; agents that induce differentiation of tumor cells (e.g., all-trans-retinoic acid); gene therapeutic reagents (e.g., antisense therapeutic reagent and nucleotide); tumor vaccines; angiogenesis inhibitors; proteasome inhibitors: NF-KB modulators; anti-CDK compounds; or HDAC inhibitors. An anticancer agent or another therapeutic technique for cancer or tumor includes a mediator that induces or stimulates apoptosis. Examples of a mediator that induces apoptosis include, but are not limited to, radiation (e.g., X-ray, gamma ray, or UV) ; tumor necrosis factor (TNF)-associated factors (e.g., TNF family receptor protein, TNF family ligand, or antibody against TRAIL, TRAIL-R1 or TRAIL-R2); kinase inhibitors (e.g., epidermal growth factor receptor (EGFR) kinase inhibitor, vascular growth factor receptor (VGFR) kinase inhibitor, fibroblast growth factor receptor (FGFR) kinase inhibitor, platelet-derived growth factor receptor (PDGFR) kinase inhibitor, and Bcr-Abl kinase inhibitor (such as GLEEVEC)); antisense molecules; antibodies (e.g., herceptin, rituxan, zevalin, and avastin); anti-estrogens (e.g., raloxifene and tamoxifen); anti-androgens (e.g., flutamide, bicalutamide, finasteride, aminoglutethamide, ketoconazole, and corticosteroid); cyclooxygenase 2 (COX-2) inhibitors (e.g., celecoxib, meloxicam, NS-398, and non-steroidal anti-inflammatory drug (NSAID)); anti-inflammatory drugs (e.g., butazolidin, decadron, deltasone, dexamethasone, dexamethasone intensol, dexon, hexadrol, hydroxychlorquine, meticorten, oradexon, orasone, oxyphenbutazone, PEDIAPRED, phenylbutazone, PLAQUENIL, prednisolone, prednisone, prelone, and tanderil); and chemotherapeutic drugs for cancer (e.g., irinotecan (CAMPTOSAR), CPT-11, fludarabine (FLUDARA), dacarbazine (DTIC), dexamethasone, mitoxantrone, mylotarg, VP-16, cisplatin, carboplatin, oxaliplatin, 5-FU, doxorubicin, gemcitabine, bortezomib, gefitinib, bevacizumab, taxotere or taxol); cellular signaling molecules; ceramides and cytokines; staurosporines; immune checkpoint inhibitors and the like.

In one embodiment, the present disclosure is for use in treating a patient for whom existing therapy was not effective. In one detailed embodiment, the present disclosure can be for use in treatment or prevention of Tumor Mutational Burden^{high} and CD8⁺ T cell^{low} inoperable or metastatic solid cancer for which there is no other therapeutic choice.

### (Medical technology such as medicaments or therapy)

A medicament for use in the technology for treatment, prevention, or prevention of recurrence of cancer of the present disclosure can be used by any approach that is known as a pharmaceutical product in the art.

In a specific embodiment, a pharmaceutical composition can comprise one or more types of compounds and at least one type of pharmaceutically acceptable carrier, wherein the one or more types of compounds can be converted into at least one type of *Mycobacterium tuberculosis* extract (i.e., prodrug) in a subject. A plurality of agents, when included, can be included in a single composition (combined agent) or in separate compositions. The agents can be formulated as a single composition using a known embodiment in the art, including those exemplified herein. A plurality of agents may be provided to achieve a therapeutic method (e.g., anticancer agent administration, radiation therapy, or the like) or with one or more other medicaments (e.g., surgery or an anticancer agent such as a chemotherapeutic agent) in addition to the immune checkpoint inhibitor and/or direct dendritic cell activating agent or means of the present disclosure. The immune checkpoint inhibitor and/or direct dendritic cell activating agent or means of the present disclosure can be provided or administered in combination with one or more other medicaments or therapeutic methods (e.g., surgery, chemotherapeutic agent, radiation therapy, or anticancer agent). The direct dendritic cell activating agent of the present disclosure can be provided or administered in combination with radiation therapy. The direct dendritic cell activating agent of the present disclosure can be provided or administered in combination with radiation therapy, an anticancer agent, or both radiation therapy and an anticancer agent. In one embodiment, one or more other medicaments or therapeutic methods (e.g., surgery, chemotherapeutic agent, radiation therapy, or anticancer agent) may be administered after an appropriate period has elapsed from administration of the immune checkpoint inhibitor and/or direct dendritic cell activating agent or means of the present disclosure. When administered separately, two or more medicaments may be provided as a kit. Non-limiting examples of anticancer agents include chemotherapeutic agents such as antimetabolites and alkylating agents, growth inhibitors, cytotoxic agents, agents used in radiation therapy, antiangiogenesis agents, apoptosis agents, anti-tubulin agents, anticancer antibiotics, anti-microtubule drugs, tyrosine kinase inhibitors, proteasome inhibitors, anaplastic lymphoma kinase inhibitors, Janus kinase inhibitors, CDK inhibitors, MEK inhibitors, Raf kinase inhibitors, PARP inhibitors, antibody drugs, other molecularly targeted drugs, platinum formulations, immunotherapy such as dendritic cell therapy, gene therapy, other low molecule drugs, other agents for treating cancer, and the like.

The compositions disclosed herein that are suitable for oral administration can be in a dosage form of a capsule, cachet, pill, tablet, lozenge (generally using a fragrance base tragacanth or acacia and sucrose), powder, granule, aqueous or non-aqueous liquid solution, aqueous or non-aqueous liquid suspension, oil-in-water emulsion, water-in-oil emulsion, elixir, syrup, troche (using inactive base such as gelatin, glycerin, sucrose, and/or acacia), and/or mouthwash, which each comprises a predetermined amount of at least one type of compound in the present disclosure.

A composition disclosed herein can be administered as a bolus, an electuary or paste.

The immune checkpoint inhibitor and/or direct dendritic cell activating agent or means of the present disclosure can be administered in any dosage form. Any dosage form, whether oral administration or parenteral administration, can be used, as long as the effect can be exerted. Preferably, parenteral administration is used.

A solid dosage form for oral administration (capsule, tablet, pill, sugar coated tablet, powder, granule, or the like) can be mixed with any of one or more types of pharmaceutically acceptable carrier such as sodium citrate or dicalcium phosphate, and/or a filler or bulking agent such as starch, lactose, sucrose, glucose, mannitol, and/or silisic acid, binding agent such as carboxymethyl cellulose, alginic acid salt, gelatin, polyvinyl pyrrolidone, sucrose, and/or acacia, a moisturizing agent such glycerol, a disintegrant such as agar, calcium carbonate, potato or tapioca starch, alginic acid, specific silicic acid salt, sodium carbonate, and sodium starch glycolate, a dissolution delaying agent such as paraffin, an absorption promotor such as a quaternary ammonium compound, humectant such as cetyl alcohol, glycerol monostearate, and polyethylene oxide-polypropylene oxide copolymer, an absorbent such as kaolin and bentonite clay, a lubricant such as talc, calcium stearate, magnesium stearate, solid polyethylene glycol, sodium lauryl sulfate, and mixture thereof, and a colorant. For a capsule, tablet, and pill, a pharmaceutical composition can also comprise a buffering agent. A similar type of solid composition can also be used as a filler in a soft and hard filled gelatin capsule using an additive such as lactose and high molecular weight polyethylene glycol.

A liquid dosage form for oral administration can comprise a pharmaceutically acceptable emulsion, microemulsion, solution, suspension, syrup, and elixir. In addition to the active ingredient, a liquid dosage form can comprise an inactive diluent used in conventional art, such as water or other solvent, solubilizing agent, and emulsifying agent, e.g., ethyl alcohol, isopropyl alcohol, ethyl carbonate, ethyl acetate, benzyl alcohol, benzyl benzoate, propylene glycol, 1,3-butylene glycol, oil (especially cotton seed oil, peanut oil, corn oil, germ oil, olive oil, castor oil, and sesame oil), glycerol, tetrahydrofuryl alcohol, polyethylene glycol, sorbitan fatty acid ester, mixture thereof, and the like. Furthermore, a compound can be dissolved using cyclodextrin such as hydroxypropyl-β-cyclodextrin.

The component of the present disclosure can comprise an adjuvant such as a humectant, emulsifying and suspending agent, sweetener, flavoring agent, colorant, fragrance, or preservative. In addition to one or more types of compounds according to the present disclosure, a suspension can comprise a suspending agent such as ethoxylated isostearyl alcohol, polyoxyethylene sorbitol and sorbitan ester, microcrystalline cellulose, aluminum methhydroxide, bentonite, agar, tragacanth, or mixture thereof.

The combination disclosed herein can be a suppository for rectal or vaginal administration, which can be prepared by mixing one or more types of compounds according to the present disclosure with one or more types of suitable non-stimulatory additives or carriers including cocoa butter, polyethylene glycol, suppository wax, salicylate, or the like. The composition is a solid at room temperature, but a liquid at body temperature. Thus, the composition melts in the rectal or vaginal cavity and releases the compound of the present disclosure. A pharmaceutical composition suitable for vaginal administration can also comprise a pessary, tampon, cream, gel, paste, foam, or spray formulation comprising a carrier known to be suitable in conventional art.

The dosage form for topical or transdermal administration of the combination of the present disclosure can comprise powder, spray, ointment, paste, cream, lotion, gel, solution, patch, and inhalant. A pharmaceutical composition or pharmaceutical tablet can be mixed with a pharmaceutically acceptable carrier and a preservative, buffering agent, or high pressure gas that may be required under aseptic conditions.

An ointment, paste, cream, and gel can comprise, in addition to the combination of the present disclosure, an additive such as animal or vegetable fat, oil, wax, paraffin, starch, tragacanth, cellulose derivative, polyethylene glycol, silicone, bentonite, silicic acid, talc, zinc oxide, or mixture thereof.

Powder or spray can comprise, in addition to the pharmaceutical composition or pharmaceutical tablet of the present disclosure, an additive such as lactose, talc, silicic acid, aluminum hydroxide, calcium silicate, or polyamide powder, or a mixture thereof. Furthermore, spray can comprise common high pressure gas such as chlorofluorohydrocarbon and volatile unsubstituted hydrocarbon such as butane and propane.

Ophthalmic formulations, optical ointment, powder, solution, and the like are also understood to be within the scope of the present disclosure.

A combination suitable for parenteral administration can comprise at least one type of pharmaceutically acceptable aseptic isotonic aqueous or non-aqueous solution, dispersant, suspension, emulsion, or aseptic powder that can be reconstituted in an aseptic injection solution or dispersant immediately before use.

The term "salt" as used herein includes acid and/or base salt formed with inorganic and/or organic acid and base. As used herein, the term "pharmaceutically acceptable salt" refers to a salt which is suitable for use in contact with tissue of a subject without excessive toxicity, stimulation, allergic reaction, and/or similar events with a reasonable balance of effect/risk ratio within the scope of a definitive medical judgment. Pharmaceutically acceptable salts are well known in conventional art. For example, pharmaceutically acceptable salts are described in detail in Berge et al., J. Pharmaceutical Sciences (1977) 66: 1-19.

Pharmaceutically acceptable salts can be produced with an inorganic or organic acid. Non-limiting examples of suitable inorganic acids include hydrochloric acid, hydrobromic acid, phosphoric acid, sulfuric acid, and perchloric acid. Non-limiting examples of suitable organic acids include acetic acid, oxalic acid, maleic acid, tartaric acid, citric acid, succinic acid, and malonic acid. Other non-limiting examples of suitable pharmaceutically acceptable salts include adipic acid salt, alginic acid salt, ascorbic acid salt, aspartic acid salt, benzenesulfonic acid salt, besilate, benzoic acid salt, bisulfuric acid salt, boric acid salt, butyric acid salt, camphoric acid salt, camphorsulfonic acid salt, citric acid salt, cyclopentanepropionic acid salt, digluconic acid salt, dodecylsulfuric acid salt, ethanesulfonic acid salt, formic acid salt, fumaric acid salt, glucoheptonic acid salt, glycerophosphoric acid salt, gluconic acid salt, hemisulfuric acid salt, heptanoic acid salt, hexanoic acid salt, hydroiodic acid salt, 2-hydroxy-ethanesulfonic acid salt, lactobionic acid salt, lactic acid salt, lauric acid salt, lauryl sulfate, malic acid salt, maleic acid salt, malonic acid salt, methanesulfonic acid salt, 2-naphthalenesulfonic acid salt, nicotinic acid salt, nitric acid salt, oleic acid salt, oxalic acid salt, palmitic acid salt, pamoic acid salt, pectinic acid salt, persulfuric acid salt, 3-phenylpropionic acid salt, phosphoric acid salt, picric acid salt, pivalic acid salt, propionic acid salt, stearic acid salt, succinic acid salt, sulfuric acid salt, tartaric acid salt, thiocyanic acid salt, p-toluenesulfonic acid salt, undecanoic acid salt, and valeric acid salt. In some embodiments, examples of organic acids that can produce a salt include acetic acid, propionic acid, glycolic acid, pyruvic acid, oxalic acid, lactic acid, trifluoroacetic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, and salicylic acid.

A salt can be prepared at the time of separation and purification of a disclosed compound or separately by reacting the compound with a suitable base or acid. Non-limiting examples of pharmaceutically acceptable salts obtained from a base include alkali metals, alkali earth metals, ammonium, and N+(C1-4 alkyl)4 salts. Non-limiting examples of suitable alkali or alkali earth metal salts include sodium, lithium, potassium, calcium, magnesium, iron, zinc, copper, manganese, and aluminum salt. Furthermore, non-limiting examples of suitable pharmaceutically acceptable salts optionally include nontoxic ammonium, quaternary ammonium, and amine cation formed using a counter ion such as a halide ion, hydroxide ion, carboxylic acid ion, sulfuric acid ion, phosphoric acid ion, nitric acid ion, lower alkyl sulfonic acid ion, and aryl sulfonic acid ion. Non-limiting examples of suitable organic bases that can produce a salt include primary amine, secondary amine, tertiary amine, substituted amine including naturally-occurring substituted amine, cyclic amine, isopropylamine, trimethylamine, diethylamine, triethylamine, tripropylamine, ethanol amine, and other basic ion exchange resins. In a specific embodiment, a pharmaceutically acceptable base addition salt can be selected from ammonium, potassium, sodium, calcium, and magnesium salt.

In an embodiment of the present disclosure, a target subject can be a patient in a state before onset of cancer, after a cancer treatment, an early stage of onset of cancer, or a precancerous condition. Alternatively, a target subject can be a healthy individual. If a healthy individual is a subject, the disclosure is performed as a preventive method.

Examples of cancer targeted in the present disclosure include, but are not limited to, esophageal cancer, gastroesophageal junction cancer, renal cell cancer, lung cancer, digestive organ cancer, leukemia, lymphoma, myeloma, brain cancer, pancreatic cancer, endometrial cancer, cervical cancer, cervical squamous cell cancer, cervical adenocarcinoma, cervical adenosquamous carcinoma, prostate cancer, liver cancer, bladder cancer, gastroesophageal adenocarcinoma, chondrosarcoma, colorectal adenocarcinoma, colorectal cancer, breast cancer, renal cell cancer, ovarian cancer, head and neck cancer, melanoma, gastric adenocarcinoma, sarcoma, urogenital cancer, gynecological cancer, adrenocortical cancer, and the like. In a specific embodiment, cancer is lung cancer. In a specific embodiment, cancer is colorectal cancer. In a specific embodiment, cancer is colorectal adenocarcinoma. In a specific embodiment, cancer is melanoma. In a specific embodiment, cancer is breast cancer. In a specific embodiment, cancer is bladder cancer. In a specific embodiment, cancer is renal cell cancer. In a specific embodiment, cancer is pancreatic cancer. In a specific embodiment, cancer is endometrial cancer. In a specific embodiment, cancer is cervical cancer. In a specific embodiment, cancer is cervical squamous cell cancer. In a specific embodiment, cancer is cervical adenocarcinoma. In a specific embodiment, cancer is cervical adenosquamous carcinoma. In a specific embodiment, cancer can be unresectable. In a specific embodiment, cancer can be progressive. In a specific embodiment, cancer can be refractory. In a specific embodiment, cancer can be recurrent. In a specific embodiment, cancer can be metastatic. In various embodiments of the present disclosure, target cancer can include normal carcinoma, carcinoma with a relatively slow progression (e.g., cancer with low sensitivity to the immune system), oral squamous cell cancer, cervical cancer, cervical squamous cell cancer, cervical adenocarcinoma, cervical adenosquamous carcinoma, MHC class I negative carcinoma on which CD8 positive T cells are generally less effective, immune checkpoint inhibitor resistant cancer, and the like. A cancer patient refers to a patient suffering from a "cancer" described above. In one embodiment, the target disease, disorder, or condition of the present disclosure comprises melanoma.

Examples of an infection targeted in the present disclosure include, but are not limited to, bacterial infections, fungal infections, parasitic protozoan infections, parasitic helminth infections, viral infections and the like. Examples of a bacterial infection include infections due to various types of bacteria such as *Streptococcus, Staphylococcus aureus, Staphylococcus epidermidis, Enterococcus, Listeria, Neisseria meningitidis, Neisseria gonorrhoeae, pathogenic Escherichia coli, Klebsiella, Proteus, Bordetella pertussis, Pseudomonas aeruginosa, Serratia, Citrobacter, Acinetobacter, Enterobacter, Mycoplasma, Clostridium, Rickettsia,* or *Chlamydia;* tuberculosis, nontuberculous mycobacteriosis, cholera, plague, diphtheria, dysentery, scarlet fever, anthrax, syphilis, tetanus, Hansen's disease, Legionella pneumonia, Leptospirosis, Lyme disease, tularemia, and Q fever. Examples of a fungal infection include aspergillosis, candidiasis, cryptococcosis, trichophytosis, histoplasmosis, and *pneumocystis* pneumonia (*carinii* pneumonia). Examples of a parasitic protozoan infection include amoebic dysentery, malaria, toxoplasmosis, leishmaniasis, and cryptosporidiosis. Examples of a parasitic helminth infection include echinococcosis, schistosomiasisjaponica, filariasis, ascariasis, and diphyllobothriasis latum. Examples of a viral infection include influenza, viral hepatitis, viral meningitis, viral gastroenteritis, viral conjunctivitis, acquired immunodeficiency syndrome (AIDS), adult T-cell leukemia, Ebola hemorrhagic fever, yellow fever, cold syndrome, rabies, cytomegalovirus infection, severe acute respiratory syndrome (SARS), Middle East respiratory syndrome (MERS), progressive multifocal leukoencephalopathy, chickenpox/herpes zoster, herpes simplex, hand-foot-and-mouth disease, dengue, Japanese encephalitis, infectious erythema, infectious mononucleosis, smallpox, rubella, acute anterior poliomyelitis (polio), measles, pharyngoconjunctival fever (pool fever), Marburg hemorrhagic fever, hantavirus renal hemorrhagic fever, Lassa fever, mumps, West Nile fever, herpangina, and chikungunya fever. In a specific embodiment, an infection is a bacterial infection. In a specific embodiment, an infection is a fungal infection. In a specific embodiment, an infection is a parasitic protozoan infection. In a specific embodiment, an infection is a parasitic helminth infection. In a specific embodiment, an infection is a viral infection.

### (Dosage and administration)

A (pharmaceutical) composition or a combination in the present disclosure can be administered at various suitable dosages and administrations. Those skilled in the art can appropriately administer the composition or combination at suitable dosage and administration in light of the description herein. For example, the composition or combination can be administered at a high frequency from the start of therapy until an effect is exerted on tumor, and the composition or combination can be administered at a low frequency after an effect is exerted on tumor. For example, a (pharmaceutical) composition or a combination in the present disclosure can be administered once a week, twice a week, three times a week or every other day, or in a combination or an appropriate modified form thereof from the start of therapy until an effect is exerted on tumor, and the composition or combination can be administered at a frequency of once every two weeks, once every three weeks or once every four weeks, at a frequency lower than said frequency, or in a combination or an appropriate modified form thereof after an effect is exerted on tumor. For more detailed administration methods, Complete Response (CR) or Partial Response (PR) according to the guidelines known to those skilled in the art, e.g., the latest RECIST guideline, or iCR or iPR or the like according to the iRECIST guideline, or an appropriate modified form thereof can be utilized.

In another exemplary example, a (pharmaceutical) composition or a combination in the present disclosure can be administered according to course therapy. For example, the composition or combination can be administered in one course consisting of 4 weeks in total, wherein the composition or combination is administered once a week for 3 weeks and the administration is stopped for 1 week. As another example of administration methods, the composition or combination can be administered in one course consisting of 8 weeks in total, wherein the composition or combination is administered once a week for 7 weeks and the administration is stopped for 1 week.

While any administration method can be utilized for a (pharmaceutical) composition or a combination in the administration methods described above, the composition or combination can be administered by subcutaneous administration in an exemplary embodiment. Although a site of administration is not limited, administration may be performed to a particular site such as, for example, upper arm.

In the present disclosure, Extract Z can be used after being diluted to 1 to 50,000-fold after manufacture. For example, Extract Z can be administered after being prepared with an undiluted solution with a saccharide content of 1.8 to 2.2 mg/mL, followed by being diluted so that one dosage would be 0.2 ug/mL to 2 mg/mL, preferably being diluted so that one dosage would be 0.2 ug/mL to 200 µg/mL, more preferably being diluted so that one dosage would be 0.2 ug/mL to 40 µg/mL. Regarding these amounts, for example, the lower limit of the dilution amount can be 0.1 µg/mL, 0.2 µg/mL, 0.3 µg/mL, 0.5 ug/mL, 1 µg/mL, 2 µg/mL, 3 µg/mL, 5 µg/mL, or 10 ug/mL while the upper limit can be 10 ug/mL, 20 ug/mL, 30 ug/mL, 50 µg/mL, 100 µg/mL, 200 µg/mL, 300 µg/mL, 500 µg/mL, 1 mg/mL, 2 mg/mL, 3 mg/mL, 5 mg/mL, 10 mg/mL, or the like. Any combination of the upper limit and the lower limit, or a combination of any values between these limits can be used.

In a non-limiting exemplary embodiment, examples of a more specific administration method and dosage include the following.
(1)
   * 20 ug/mL of an Extract Z undiluted solution in terms of D-arabinose may be subcutaneously administered at 1 mL per dose, once daily, and twice a week from the day of the start of radiation therapy to the day of the end of the radiation therapy (up to 8 weeks).
(2)
   * For the first 3 years, A solution (2 ug/mL of an Extract Z undiluted solution in terms of D-arabinose) and B solution (0.2 ug/mL of an Extract Z undiluted solution in terms of D-arabinose) may be alternatively administered subcutaneously at 1 mL per dose every other day (or three times a week). If there is no recurrence or metastasis, administration can be performed twice a week in the next 2 years, and if there is no abnormality, administration can be reduced to once a week and can be appropriately terminated. Administration can be performed every other day (or three times a week) while the lesion continues.
(3)
   * Embodiment C: 0.2 ug/mL of an Extract Z undiluted solution in terms of D-arabinose can be subcutaneously administered to the upper arm at 1 mL per dose, once daily, and twice a week (at an interval of 3 to 4 days) from the day of the start of radiation therapy to day 28 after the end of the radiation therapy. Subsequently, 0.2 ug/mL of an Extract Z undiluted solution in terms of D-arabinose can be subcutaneously administered to the upper arm at 1 mL per dose, once daily, and once every two weeks until progression/recurrence/metastasis are observed.
   *Embodiment D: 0.2 µg/mL, 2 µg/mL, or 20 ug/mL of an Extract Z undiluted solution in terms of D-arabinose can be subcutaneously administered to the upper arm at 1 mL per dose, once daily, and twice a week (at an interval of 3 to 4 days) from the day of the start of radiation therapy to day 28 after the end of the radiation therapy. Subsequently, 0.2 ug/mL, 2 ug/mL, or 20 ug/mL of an Extract Z undiluted solution in terms of D-arabinose can be subcutaneously administered to the upper arm at 1 mL per dose, once daily, and once every two weeks until progression/recurrence/metastasis are observed.
   * Embodiment E: 0.2 ug/mL of an Extract Z undiluted solution in terms of D-arabinose can be subcutaneously administered to the upper arm at 1 mL per dose, once daily, and twice a week (at an interval of 3 to 4 days) from the day of the start of radiation therapy to day 28 after the end of the radiation therapy. Subsequently, 0.2 ug/mL of an Extract Z undiluted solution in terms of D-arabinose can be subcutaneously administered to the upper arm at 1 mL per dose, once daily, and once every two weeks until progression/recurrence/metastasis are observed.
   * Embodiment F

Examples of preventive therapy targeting a healthy adult can include the following.
* (Intermittently administered group) 0.02 ug/mL, 0.2 ug/mL, 2 ug/mL, or 40 ug/mL of an Extract Z undiluted solution in terms of D-arabinose can be subcutaneously administered to the upper arm once daily and at an interval of 2 days for 13 days from day 1, the day of the start of the administration, to day 13 (five times of administration in total).
* (Daily administered group) 0.02 ug/mL, 0.2 ug/mL, 2 ug/mL, or 40 ug/mL of an Extract Z undiluted solution in terms of D-arabinose can be subcutaneously administered to the upper arm once daily and every day for 13 days from day 1, the day of the start of the administration, to day 13.
* Embodiment G: 0.2 ug/mL or 40 ug/mL of an Extract Z undiluted solution in terms of D-arabinose can be subcutaneously administered to the upper arm at 1 mL per dose, once daily, and twice a week (at an interval of 3 to 4 days) from the day of the start of radiation therapy to day 28 after the end of the radiation therapy. Subsequently, 0.2 ug/mL or 40 ug/mL of an Extract Z undiluted solution in terms of D-arabinose can be subcutaneously administered to the upper arm at 1 mL per dose, once daily, and once every two weeks for 2 or more years or until progression/recurrence/metastasis are observed.
* Embodiment H: 2 µg/mL, 20 µg/mL, or 40 ug/mL of an Extract Z undiluted solution in terms of D-arabinose can be subcutaneously administered to the upper arm at 1 mL per dose, once daily, and twice a week (at an interval of 3 to 4 days) from the day of the start of radiation therapy to the day of the end of the radiation therapy. Subsequently, 2 µg/mL, 20 µg/mL, or 40 ug/mL of an Extract Z undiluted solution in terms of D-arabinose can be subcutaneously administered to the upper arm at 1 mL per dose, once daily, and once every two weeks for 1 or more year or until progression/recurrence/metastasis are observed.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on the Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### [Examples]

The Examples are described hereinafter. When necessary, animals used in the following Examples were handled in compliance with relevant ethical standards and guidelines, based on the Declaration of Helsinki. While the specific products described in the Examples were used, reagents can be substituted with an equivalent product from another manufacturer (Sigma-Aldrich, Wako Pure Chemical, Nacalai Tesque, R & D Systems, USCN Life Science INC, or the like).

The manufacturers and catalogue numbers of the antibodies and staining reagents used in the following Examples are as follows.
CD80 (Miltenyi Biotec, catalogue number 130-102-372)
CD86 (Miltenyi Biotec, catalogue number 130-102-506)
CD11b (Miltenyi Biotec, catalogue number 130-113-811)
CD11c (Miltenyi Biotec, catalogue number 130-122-016)
CD45 (Miltenyi Biotec, catalogue number 130-119-130)
CD4 (Miltenyi Biotec, catalogue number 130-123-899)
CD8 (Life Technologies Corporation, catalogue number 25-0081-82)
TCRβ (BioLegend Incorporated, catalogue number 109220)
NK (anti-CD49b antibody; Miltenyi Biotec, catalogue number 130-102-258)
MHC class II (Miltenyi Biotec, catalogue number 130-123-785)
PD-L1 (anti-CD274 antibody; Life Technologies Corporation, catalogue number 12-5982-81)
PI (Propidium Iodide Solution; dead cell marker; Miltenyi Biotec, catalogue number 130-093-233)
CD28 (Miltenyi Biotec, catalogue number 130-111-973)
CD62L (Miltenyi Biotec, catalogue number 130-102-543)
CD44 (Miltenyi Biotec, catalogue number 130-102-511)
Treg (CD25⁺CD4⁺FoxP3⁺) (Miltenyi Biotec, catalogue number 130-120-674)
Zombie Green (dead cell marker for intracellular staining)
(BioLegend Incorporated, catalogue number 423111)

### (Manufacturing Example: Extract Z)

Extract Z used in this Example was manufactured in the following manner.

Human *Mycobacterium tuberculosis* strain Aoyama B which had been lyophilized and stored (-20°C) was subjected to seed culture at 37 ± 1°C in a Sauton potato medium^{(l)}.

The cultured bacteria were transferred to a production medium⁽²⁾ and cultured (primary culture) for 5 to 7 weeks at 37 ± 1°C. The resulting cells were washed with water for injection. To the cells, water for injection was then added in an amount 20-fold of the weight of the wet cells. The mixture was heated at 100°C for 120 minutes to obtain an extract. The extract was filtered with a 0.45 µm-membrane filter and then concentrated under reduced pressure so that the saccharide content (in terms of D-arabinose by the phenol-sulfuric acid method) would be 4.0 to 6.0 mg/mL to obtain a concentrate. Subsequently, in order to remove proteins, 1 W/V% of sulfosalicylic acid was added to the concentrate. The mixture was left standing for 15 to 20 minutes at 10°C or lower. Precipitates were then removed by centrifugation (10°C or lower, 1,150 × G, 10 minutes) to recover the supernatant. The protein concentration of the supernatant was 0.30 mg/mL or lower (Lowry method, in terms of tyrosine) . The supernatant was further processed to remove sulfosalicylic acid until the concentration was at or below the detection limit (10 ppm or less, method using ferric chloride solution). The resultant solution was concentrated under reduced pressure so that the saccharide content would be 1.8 to 2.2 mg/mL, and the concentrate was combined with sodium chloride (0.9 W/V%) and cold ethanol at the same volume as the concentrate. The mixture was left standing for 40 hours or longer at 10°C or lower, and then the precipitates (polysaccharide of high molecular weight region) were removed by centrifugation (10°C or lower, 2,040 × G, 10 minutes). Subsequently, the supernatant was combined with four times the amount of cold ethanol, and the mixture was left standing for 40 hours or longer at 10°C or lower and centrifuged (10°C or lower, 2,040 × G, 10 minutes) to recover precipitates. The precipitates were dissolved in water for injection. After the saccharide content was adjusted to 1.8 to 2.2 mg/mL, the solution was filtered with a 0.45 um membrane filter and sterilized with high pressure steam (121°C, 20 minutes) to prepare an Extract Z solution.

### (1) Sauton-potato medium

Washed potato slices were soaked in a Sauton medium, sterilized for 15 minutes at 115°C, and then used as a Sauton-potato medium.

### Sauton Medium

L-asparagine (monohydrate) 4.0 g
Citric acid (monohydrate) 2.0 g
Magnesium sulfate (heptahydrate) 0.5 g
Potassium monohydrogenphosphate (anhydride) 0.5 g
Ammonium iron citrate 0.05 g
Glycerol 60 ml

The above ingredients were dissolved in water to prepare a 1,000 ml solution. pH was adjusted to 7.0 to 7.3 by using a sodium hydroxide solution.

### (2): Production medium

L-asparagine (monohydrate) 4.0 g
Citric acid (monohydrate) 2.0 g
Magnesium sulfate (heptahydrate) 0.5 g
Potassium monohydrogenphosphate (anhydride) 0.5 g
Ammonium iron citrate 0.05 g
Glycerol 60 ml

The above ingredients were dissolved in water to prepare a 1,000 ml solution and sterilized with high pressure steam (121°C, 20 minutes). pH was adjusted to 7.0 to 7.3 by using a sodium hydroxide solution.

The physicochemical properties of the resulting Extract Z solution were as follows.
(1) Appearance:
   Pale yellow clear liquid
(2) pH:
   4.50 to 5.30
(3) Protein content:
   3.5 wt.% (as an amino acid) in a lyophilized product
(4) Nucleic acid content:
   0.1 wt.% in a lyophilized product
(5) Primary constituent monosaccharides of polysaccharide:
   Mannose 43.4 wt.%, arabinose 18.2 wt.%, and glucose 10.4 wt.% (hydrolyzed with 2N trifluoroacetic acid for two hours at 100°C, and then subjected to liquid chromatography using 2-cyanoacetamide fluorescent derivative (S. Honda, et al., Anal. Chem., 52, 1079 (1980)).

The Extract Z solution prepared by the method described in the Manufacturing Example described above can be appropriately diluted prior to use. In the following Examples, the Extract Z solution was diluted 1 to 50,000-fold and adjusted to a suitable concentration for use.

### (Example 1: Example of treatment or prevention of Tumor Mutational Burden^{high} and CD8⁺ T cell^{low} inoperable or metastatic solid cancer for which there is no other therapeutic choice, wherein the treatment or prevention comprises an immunoadjuvant (e.g., a Mycobacterium tuberculosis extract-related substance))

This Example shows demonstration of treatment or prevention of Tumor Mutational Burden^{high} and CD8⁺ T cell^{low} inoperable or metastatic solid cancer for which there is no other therapeutic choice, wherein the treatment or prevention comprises an immunoadjuvant.

Saline or Extract Z is administered to a group of patients who have solid tumor, have no existing therapeutic choice, and are Tumor Mutational Burden^{high} and CD8⁺ T Cell invasion^{low}. An immune checkpoint inhibitor (e.g., pembrolizumab) is administered at the same time as or at a different time from saline or Extract Z. Subsequently, PFS (Progression-Free Survival), OS (Overall Survival), and ORR (Objective Response Rate) in the group of patients are measured.

### (Example 2: Example of enhancement of CXCL10 production by an immunoadjuvant)

This Example demonstrates enhancement of CXCL10 production by the agent of the present disclosure.

Bone marrow-derived cells were collected from a C3H/HeJ mouse (male), and the collected bone marrow-derived cells were cultured at 4 × 10⁶/plate for 6 days (in the presence of 20 ng/mL GM-CSF and 20 ng/mL IL-4). The bone marrow-derived cells after culture were collected, seeded at 2 × 10⁵/well, and stimulated with 0.4 µg/mL, 0.8 µg/mL, 1.6 µg/mL, and 3.2 ug/mL of Extract Z and TLR2 ligand (Pam3csk4, Heatkilled *Mycobacterium tuberculosis*). The culture supernatant was collected after 6 hours from the stimulation, and the concentration of CXCL10 was measured by ELISA method.

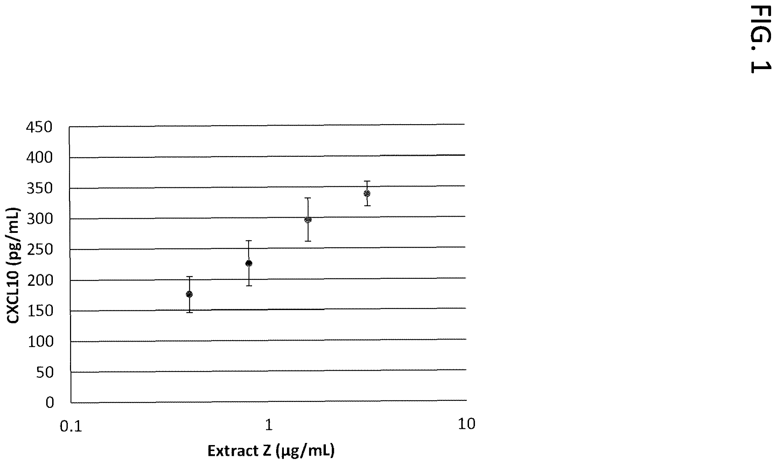

Figure 1 shows the result. An increase in the concentration of CXCL10 in the culture supernatant was observed as the concentration of Extract Z used for stimulation was increased. Thus, it was found that Extract Z has an action of enhancing CXCL10 production.

### (Example 3: Example of treatment of cancer or tumor that is CXCL10 positive by an immunoadjuvant)

### (CXCL10 positive)

An immunoadjuvant is administered to a model subcutaneously transplanted with cancer cells to confirm the antitumor effect or life-prolonging effect. Tumor is collected from a subcutaneously transplanted model in which the antitumor effect or life-prolonging effect was observed, and the amount of expression of the CXCL10 gene in the tumor is studied to confirm that the amount of expression is high.

### (CXCR3 positive)

An immunoadjuvant is administered to a model subcutaneously transplanted with cancer cells to confirm the antitumor effect or life-prolonging effect. Tumor is collected from a subcutaneously transplanted model in which the antitumor effect or life-prolonging effect was observed, and the CXCR3 positive cells in the tumor are studied by a flow cytometer or immunostaining to confirm that the positive cell proportion is high.

### (Example 4: Example of the effect of promoting infiltration of CD8⁺ T cells into tumor)

This Example shows the effect of the agent of the present disclosure to promote infiltration of CD8⁺ T cells into tumor.

Saline or 1 mg/kg of Extract Z was subcutaneously administered to the right inguinal region of a C3H/HeN mouse once daily (40 mice for each group) . 1 × 10⁶ oral squamous cell carcinoma Sq-1979 cells were subcutaneously infused on the ventral side on day 29 after starting the administration of saline or Extract Z. Tumor was collected after 7 days from introduction of Sq-1979 cells, the samples of 5 mice were pooled, and measurement was performed using a flow cytometer.

### (Antibody and reagent used)

Antibody against CD45
Antibody against CD8
Antibody against TCRβ chain
PI (dead cell marker)

### (Result)

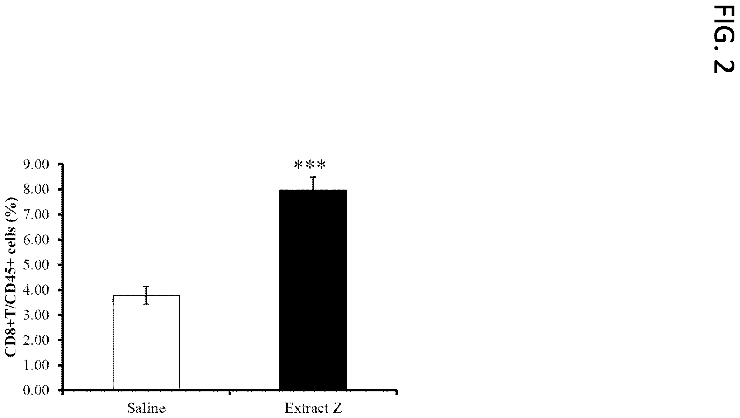

Figure 2 shows the result. Administration of Extract Z resulted in an increase in CD8⁺ T cells in tumor. Meanwhile, since the action of Extract Z is non-specific immunostimulatory, CD8⁺ T cells infiltrating into tumor include both cells having the antitumor effect and cells that express CTLA-4 and inhibit the antitumor effect.

### (Example 5: Synergistic effect of a combination of Extract Z and another anticancer agent (1))

Saline or Extract Z is administered to a group of patients who have non-small cell lung cancer, have no existing therapeutic choice, and are EGFR mutation positive and EGFR-TKI intolerant/refractory. Administration of an immune checkpoint inhibitor and palliative radiation therapy are performed at the same time as or at a different time from saline or Extract Z. Subsequently, PFS (Progression-Free Survival), OS (Overall Survival), and ORR (Objective Response Rate) in the group of patients are measured.

### (Example 6: Example showing the proportion of tumor infiltrating cells as a result of administration of Extract Z)

This Example shows analysis of the proportion of tumor infiltrating cells as a result of administration of the agent of the present disclosure.

Saline or 1 mg/kg of Extract Z was repeatedly and subcutaneously administered to the right inguinal region of a C3H/HeN mouse once daily for 35 days (20 mice for each group). For Table 1, 1 × 10⁶ oral squamous cell carcinoma Sq-1979 cells were subcutaneously infused on the ventral side on day 29 after starting the administration of saline or Extract Z, and 200 µg/body of anti-PD-1 antibody was intraperitoneally infused on day 28, day 31, and day 34 after starting the administration of saline or Extract Z (Table 1). For Table 2, 1 × 10⁶ oral squamous cell carcinoma Sq-1979 cells were subcutaneously infused on the ventral side on day 29 after starting the administration of saline or Extract Z, and 200 µg/body of anti-PD-1 antibody or Control antibody was intraperitoneally infused on day 28, day 31, and day 34 after starting the administration of saline or Extract Z.

On day 36 after starting the administration of saline or Extract Z, tumor and lymph nodes were collected, the collected samples of 5 mice were pooled, and measurement was performed using a flow cytometer.

### (Antibody and reagent used)

Antibodies against CD45, CD4, CD8, and TCR (T cell receptor)
Antibodies against NK, MHC class II, and PD-L1
PI (dead cell marker)

### (Result)

Table 1 and Table 2 show the results. While the percentage of CD8⁺ T cells in tumor infiltrating cells is 4.34% in the saline administered group, the percentage of CD8⁺ T cells is 10.43% in the Extract Z administered group (Table 1). This result demonstrates that administration of Extract Z induces infiltration of CD8⁺ T cells into tumor. While the percentage of CD8⁺ T cells/CD45⁺ T cells is 4.82% in the saline administered group, the percentage of CD8⁺ T cells/CD45⁺ T cells is 10.54% in the Extract Z administered group. Furthermore, while the group administered with an anti-PD-1 antibody alone shows 3.91%, the group administered with both Extract Z and an anti-PD-1 antibody shows 11.58% (Table 2).

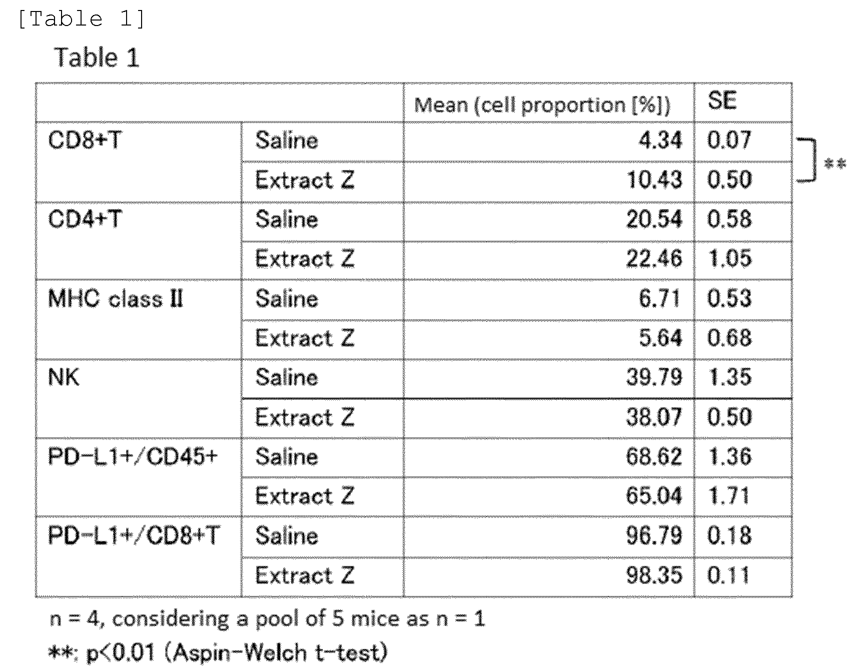

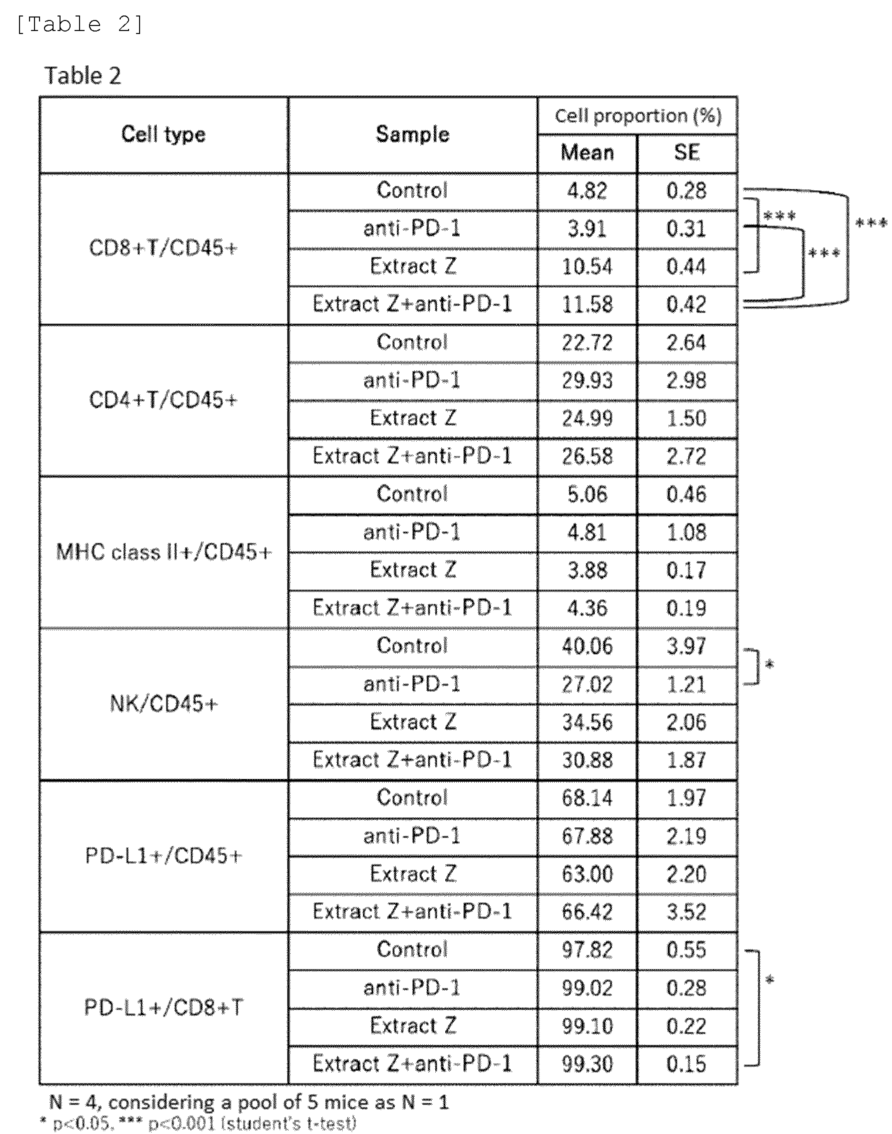

### (Example 7: Example showing the proportion of antigen-presenting cell markers in a lymph node as a result of administration of Extract Z)

This Example shows analysis of the proportion of antigen-presenting cell markers in a lymph node as a result of administration of the agent of the present disclosure.

Saline or 1 mg/kg of Extract Z was repeatedly and subcutaneously administered to the right inguinal region of a C3H/HeN mouse once daily for 35 days (20 mice for each group). 1 × 10⁶ oral squamous cell carcinoma Sq-1979 cells were subcutaneously infused on the ventral side on day 29 after starting the administration of saline or Extract Z, and 200 µg/body of anti-PD-1 antibody or Control antibody was intraperitoneally infused on day 28, day 31, and day 34 after starting the administration of saline or Extract Z.

On day 36 after starting the administration of saline or Extract Z, tumor and lymph nodes were collected, the collected samples of 5 mice were pooled, and measurement was performed using a flow cytometer.

### (Antibody and reagent used)

Antibodies against CD45, CD80, CD86, CD11b, CD11c, MHC class II, and PD-L1
PI (dead cell marker)

### (Result)

Tables 3 and 4 show the results. While the percentage of CD80⁺/MHC class II cells in antigen-presenting cell markers in lymph nodes is 6.09% in the saline administered group, the percentage of CD80⁺/MHC class II cells is 8.38% in the Extract Z administered group (Table 3). This result demonstrates that administration of Extract Z enhances CD80⁺ expression. While the percentage of CD80⁺/MHC class II cells is 6.08% in the group administered with an anti-PD-1 antibody, the percentage of CD80⁺/MHC class II cells is 6.89% in the group administered with both Extract Z and an anti-PD-1 antibody (Table 4). Thus, a tendency of administration of Extract Z inducing CD80⁺ cell expression was observed. From these results, it is considered that CD80⁺ cell expression may be the result of Extract Z alone, not the additive and synergistic effect of Extract Z and an anti-PD-1 antibody.

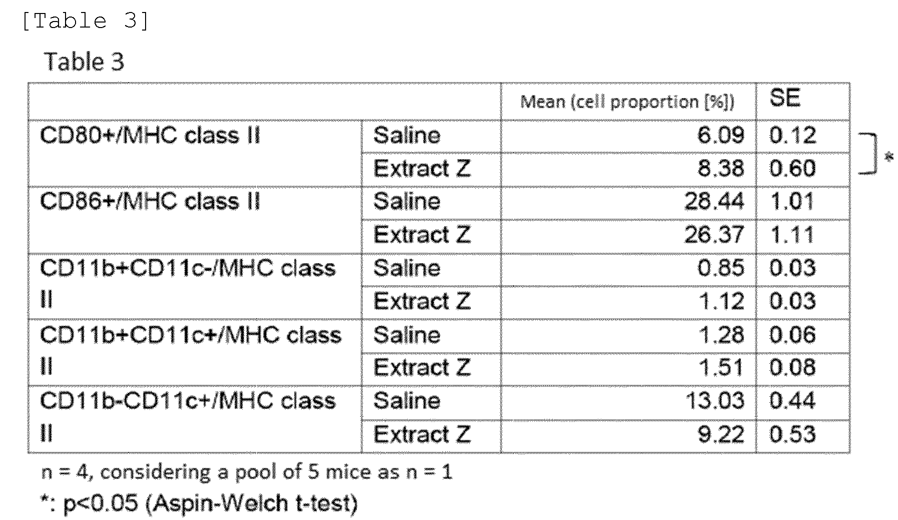

### [Table 4]

**Table 4**

| **Cell type** | **Sample** | Cell proportion (%) | |
|---|---|---|---|
| | | **Mean** | **SE** |
| CD80+/MHC class II | Saline + anti-PD-1 | 6.08 | 0.27 |
| | Extract Z + anti-PD-1 | 6.89 | 0.22 |
| CD86+/MHC class II | Saline + anti-PD-1 | 25.90 | 0.82 |
| | Extract Z + anti-PD-1 | 24.65 | 1.78 |
| CD11b+CD11c-/ MHC class II | Saline + anti-PD-1 | 1.18 | 0.11 |
| | Extract Z + anti-PD-1 | 1.41 | 0.06 |
| CD11b+CD11c+/ MHC class II | Saline + anti-PD-1 | 0.84 | 0.06 |
| | Extract Z + anti-PD-1 | 0.86 | 0.04 |
| CD11b-CD11c+/ MHC class II | Saline + anti-PD-1 | 10.51 | 0.50 |
| | Extract Z + anti-PD-1 | 9.76 | 0.61 |

| | | | |
|---|---|---|---|
| **N = 4, considering a pool of 5 mice as N = 1** | | | |

### (Example 8: Example showing the proportion of major cells in a lymph node as a result of administration of Extract Z)

This Example shows analysis of the proportion of major cells in a lymph node as a result of administration of the agent of the present disclosure.

Saline or 1 mg/kg of Extract Z was repeatedly and subcutaneously administered to the right inguinal region of a C3H/HeN mouse once daily for 35 days (20 mice for each group). 1 × 10⁶ oral squamous cell carcinoma Sq-1979 cells were subcutaneously infused on the ventral side on day 29 after starting the administration of saline or Extract Z, and 200 µg/body of anti-PD-1 antibody or Control antibody was intraperitoneally infused on day 28, day 31, and day 34 after starting the administration of saline or Extract Z.

On day 36 after starting the administration of saline or Extract Z, lymph nodes were collected, the collected samples of 5 mice were pooled, and measurement was performed using a flow cytometer.

### (Antibody and reagent used)

Antibodies against CD45, CD4, CD8, and TCR (T cell receptor)
Antibodies against NK, MHC class II, and PD-L1
PI (dead cell marker)

### (Result)

Table 5 shows the results. While the percentage of CD8⁺ T cells in major cells in lymph nodes is 20.70% in the saline administered group, the percentage of CD8⁺ T cells is 22.38% in the Extract Z administered group. Furthermore, while the percentage of CD8⁺ T cells is 21.55% in the group administered with an anti-PD-1 antibody alone, the percentage of CD8⁺ T cells is 23.56% in the group administered with both Extract Z and an anti-PD-1 antibody. These results demonstrate that administration of Extract Z enhances accumulation of CD8⁺ T cells in lymph nodes.

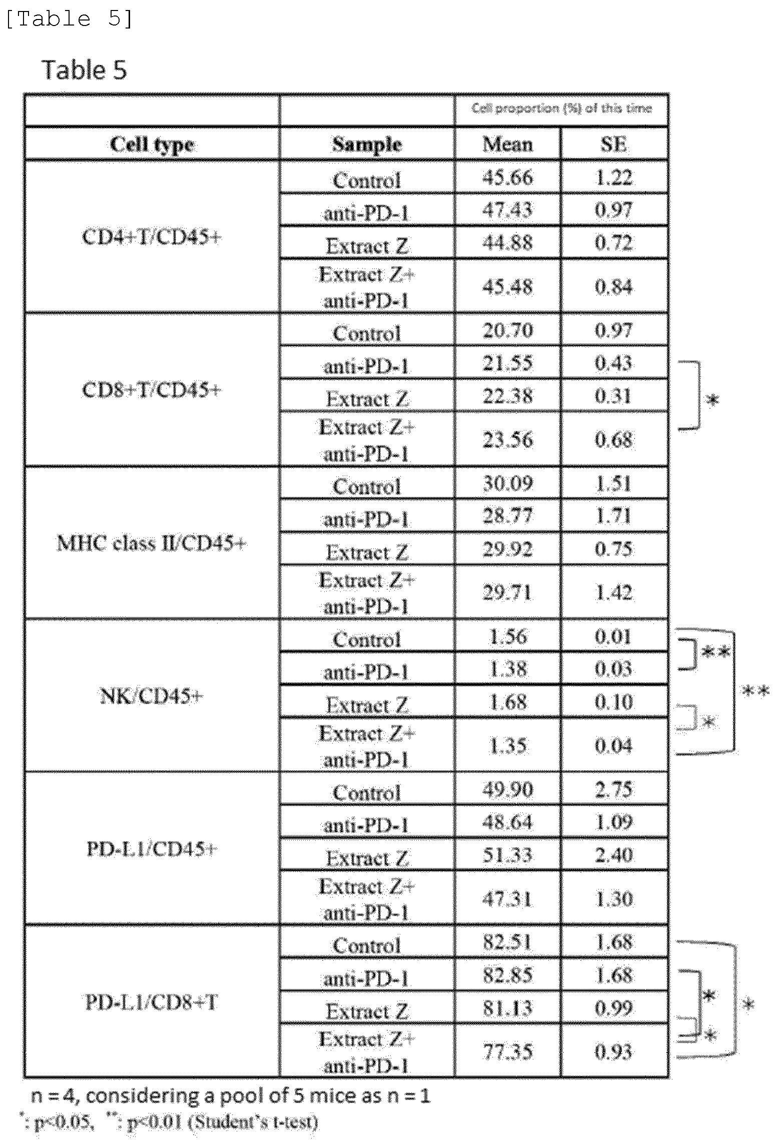

### (Example 10: Example showing the proportion of CD8 T cell activating markers in a lymph node as a result of administration of Extract Z)

This Example shows analysis of the proportion of CD8 T cell activating markers in a lymph node as a result of administration of the agent of the present disclosure.

Saline or 1 mg/kg of Extract Z was repeatedly and subcutaneously administered to the right inguinal region of a C3H/HeN mouse once daily for 35 days (20 mice for each group). 1 × 10⁶ oral squamous cell carcinoma Sq-1979 cells were subcutaneously infused on the ventral side on day 29 after starting the administration of saline or Extract Z, and 200 µg/body of anti-PD-1 antibody or Control antibody was intraperitoneally infused on day 28, day 31, and day 34 after starting the administration of saline or Extract Z.

On day 36 after starting the administration of saline or Extract Z, lymph nodes were collected, the collected samples of 5 mice were pooled, and measurement was performed using a flow cytometer.

### (Antibody and reagent used)

Antibodies against CD45, CD4, CD8, CD28, CD62L, CD44, and TCR (T cell receptor)
PI (dead cell marker)

### (Result)

Table 6 shows the results. While the percentage of effector memory CD8⁺ T cells in CD8 T cells in lymph nodes is 6.16% in the saline administered group, the percentage of effector memory CD8⁺ T cells is 8.38% in the Extract Z administered group. Furthermore, while the percentage of effector memory CD8⁺ T cells is 5.37% in the group administered with an anti-PD-1 antibody alone, the percentage of effector memory CD8⁺ T cells is 7.97% in the group administered with both an anti-PD-1 antibody and Extract Z. This result demonstrates that administration of Extract Z induces effector memory CD8⁺ T cells.

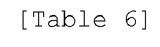

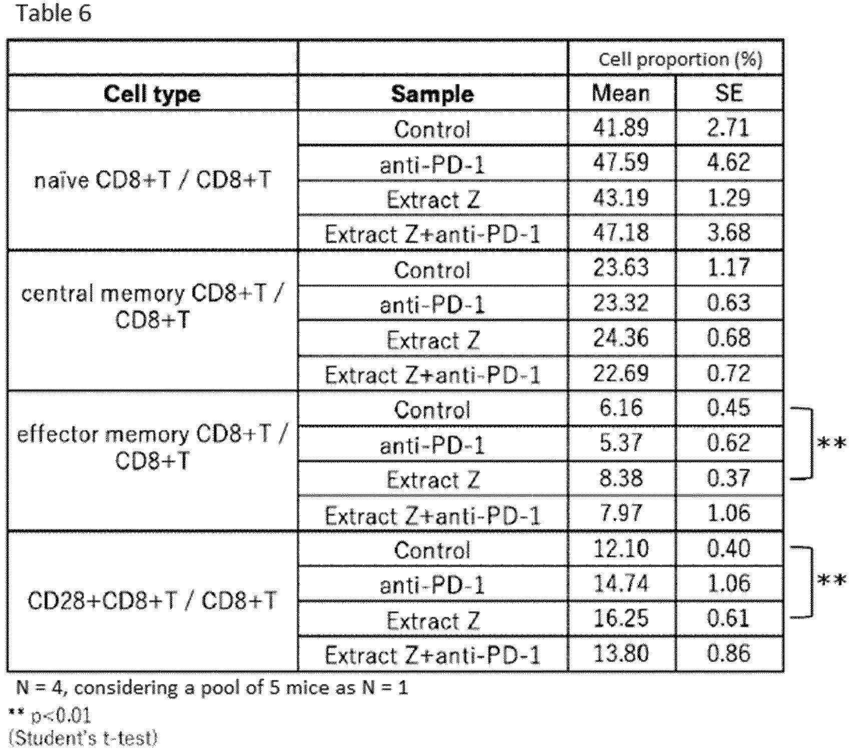

### (Example 11: The effect of combined use of Extract Z and an immune checkpoint inhibitor)

In this Example, Extract Z and an anti-CTLA-4 antibody are administered to a mouse tumor model to confirm that the antitumor effect or life-prolonging effect synergistically increases.

A splenocyte or lymph node is collected from a mouse, and an immune cell is isolated and Extract Z and an anti-CTLA-4 antibody are added thereto. Extract Z exhibits an IFN-γ production action (T cell activation action) by being added *in vitro.* Thus, it is confirmed whether the IFN-γ production synergistically increases by combined use with a CTLA-4 antibody.

### (Example 12: Example showing that a CD8⁺ T cell infiltrating into tumor increases by administration of Extract Z)

This Example confirms CTLA-4 expression in a tumor infiltrating CD8⁺ T cell which is found as a result of administration of Extract Z.

It is confirmed that administration of Extract Z not only promotes infiltration of overall CD8⁺ T cells but also promotes infiltration of CTLA-4⁺ CD8⁺ T cells that inhibit antitumor effect.

### (Example 13: Example in which the immunoadjuvant is administered in combination with other medicaments (not limited to an immune checkpoint inhibitor))

Saline or Extract Z is administered to a group of patients with head and neck cancer (oral cavity/oropharynx/hypopharynx/larynx) at a stage for which chemical radiation therapy is suitable. Administration of a platinum formulation (cisplatin or carboplatin) and radical radiation irradiation are performed at the same time as or at a different time from saline or Extract Z. Subsequently, PFS (Progression-Free Survival), OS (Overall Survival), and ORR (Objective Response Rate) in the group of patients are measured.

### (Example 14: Examples of clinical trials with a Mycobacterium tuberculosis hot water extract and chemical radiation therapy for cervical cancer)

Saline or Extract Z is administered to a group of patients with cervical cancer at a stage for which chemical radiation therapy is suitable. Administration of a platinum formulation (cisplatin or carboplatin) and radical radiation irradiation are performed at the same time as or at a different time from saline or Extract Z. Subsequently, immune parameters (CD11c, CD14, CD16, CD19, CD24, CD27, CD38, CD80, CD86, CD123, CD138, CCR5, CCR7, CXCR3, HLA-DR, CD3, CD4, CD8, CD45RA, CD56, CD69, CD159a, CTLA-4, NKp46, PD-1, IFNγ, TNFα, perforin, granzyme B, IL4, FoxP3, IL17A, Ki67, CXCL9, CXCL10, IL10, IL12p70), PFS (Progression-Free Survival), OS (Overall Survival), and ORR (Objective Response Rate) in the group of patients are measured.

### (Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2020-171491 filed on October 9, 2020 with the Japan Patent Office, and Japanese Patent Application No. 2020-217698 filed on December 25, 2020 with the Japan Patent Office. It is understood that the entire content thereof is incorporated herein by reference.

### [Industrial Applicability]

The present disclosure provides a method of preventing and treating a disease such as cancer based on a conventionally unavailable mechanism.

## Claims

1. A composition for use in activation of CXCL10 production, comprising an immunoadjuvant.

2. The composition of claim 1, wherein the activation of CXCL10 production is in a cell comprising a bone marrow-derived cell.

3. The composition of claim 1, wherein the activation of CXCL10 production is in an antigen-presenting cell.

4. The composition of claim 1, wherein the activation of CXCL10 production is in a lymph node.

5. A composition for use in treating cancer or tumor that is CXCL10 positive, comprising an immunoadjuvant.

6. A composition for use in promoting infiltration of a CXCR3 positive cell into cancer or tumor, comprising an immunoadjuvant.

7. A composition for use in promoting infiltration of a CD8 positive T cell into cancer or tumor, comprising an immunoadjuvant.

8. A composition for use in activating an immune cell in a lymph node, comprising an immunoadjuvant.

9. A composition for use in promoting infiltration of a CD8 positive T cell into a lymph node, comprising an immunoadjuvant.

10. The composition of claim 9, wherein the CD8 positive T cell is an effector memory CD8⁺ T cell.

11. A pharmaceutical composition comprising an immunoadjuvant, **characterized in that** the composition is used with an anticancer agent or another therapeutic technique for cancer or tumor.

12. A combination medicament of: an anticancer agent or another therapeutic technique for cancer or tumor; and an immunoadjuvant.

13. The pharmaceutical composition of claim 11 or the combination medicament of claim 12 for use in treating a patient for whom existing therapy was not effective.

14. The pharmaceutical composition of claim 11 or the combination medicament of claim 12 for use in treatment or prevention of Tumor Mutational Burden^{high} and CD8⁺ T cell^{low} inoperable or metastatic solid cancer for which there is no other therapeutic choice, the pharmaceutical composition or combination medicament comprising an immunoadjuvant.

15. The composition, pharmaceutical composition, or combination medicament of any one of claims 1 to 14, wherein the immunoadjuvant comprises a *Mycobacterium tuberculosis* extract or a portion thereof.

16. The composition, pharmaceutical composition, or combination medicament of claim 14, wherein the immunoadjuvant comprises a human *Mycobacterium tuberculosis* hot water extract.

17. The pharmaceutical composition of claim 11 or the combination medicament of claim 12 for use in treatment or prevention of Tumor Mutational Burden^{high} and CD8⁺ T cell^{low} inoperable or metastatic solid cancer for which there is no other therapeutic choice, the pharmaceutical composition or combination medicament comprising an immunoadjuvant, wherein the immunoadjuvant comprises a *Mycobacterium tuberculosis* extract or a portion thereof.

18. The pharmaceutical composition or combination medicament of claim 16, wherein the immunoadjuvant comprises a human *Mycobacterium tuberculosis* hot water extract.
